# EUROPEAN PATENT APPLICATION

(11) **EP 4 579 679 A1**
(43) Date of publication of application: **02.07.2025**
(21) Application number: 24223571.1
(22) Date of filing: 28.12.2024
(51) Int. Cl.: G16H 40/20, G16H 40/67

(54) **CONTROL SYSTEM FOR OPERATING ROOM AND METHOD FOR CONTROLLING CONNECTION OF OPERATING ROOM**

(30) Priority: 29.12.2023 CN 202311865383
(71) Applicant: Nanjing Mindray Bio-Medical Electronics Co., Ltd., Nanjing, Jiangsu 211111 (CN)
(72) Inventor: DING, Rui, Nanjing, 211111 (CN); WANG, Dan, Nanjing, 211111 (CN)
(74) Representative: KIPA AB

(57) **Abstract**

Disclosed are a control system for an operating room, and a method for controlling connection of an operating room. The system includes a first control apparatus, which is in communicative connection with a medical device and with a position changes synchronously with a position of the medical device; and a second control apparatus which is arranged at a designated position of the operating room. The first control apparatus includes a first scanning module, the second control apparatus includes a second scanning module, for obtaining a scanning result which indicates a distance, a signa strength or a position. When the scanning result satisfies a first condition, establish a communicative connection between the medical device and the control device. When the scanning result satisfies the first condition or a second condition, maintain a communicative connection between the medical device and the control device. A stability of the communicative connection is improved.

## Description

### CROSS-REFERENCE TO RELATED APPLICATION

This disclosure claims priority to Chinese patent Application No. 202311865383.4, filed on December 29, 2023, the content of which is incorporated herein by reference in its entirety.

### TECHNICAL FIELD

The disclosure relates to a medical field, and more particularly to a control system for an operating room, and a method for controlling connection of an operating room.

### BACKGROUND

Medical devices in an operating room are connected with a control device for the operating room through wired or wireless communication. In this way, during an operating process, it is necessary to control working parameters of each medical device uniformly through the control device. Before establishing a communicative connection, regardless of wired or wireless communication is employed, medical staff need implement manual configuration to establish the communicative connection.

### SUMMARY

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

A first aspect according to an embodiment of this disclosure provides a control system for an operating room, including: a control device for the operating room, a first control apparatus, and a second control apparatus;
the first control apparatus is in communicative connection with a medical device, wherein the first control apparatus includes a first scanning module, and a position of the first control apparatus changes synchronously with a position of the medical device;
the second control apparatus is arranged at a designated position of the operating room inside which room the control device is located, wherein the second control apparatus includes a second scanning module; the first scanning module is configured to receive a scanning signal which is transmitted by the second scanning module and/or the second scanning module is configured to receive a scanning signal which is transmitted by the first scanning module, so as to obtain a scanning result;
wherein:
   when the scanning result satisfies a first condition and no communicative connection has been established between the medical device and the control device, a communicative connection is established between the medical device and the control device;
   when the scanning result does not satisfy the first condition and no communicative connection has been established between the medical device and the control device, no communicative connection between the medical device and the control device is maintained;
   when the scanning result satisfies the first condition or satisfies a second condition and a communicative connection has been established between the medical device and the control device, said communicative connection is maintained between the medical device and the control device;
   when the scanning result does not satisfy the first condition and the second condition and a communicative connection has been established between the medical device and the control device, said communicative connection between the medical device and the control device is disconnected;
   wherein, when the scanning result indicates a distance, a maximum distance which satisfies the first condition, is less than a maximum distance which satisfies the second condition; or
   when the scanning result indicates a strength of the scanning signal, a minimum signal strength which satisfies the first condition is greater than a minimum signal strength which satisfies the second condition; or
   when the scanning result indicates a position of the first scanning module, an area which corresponds to a possible position of the first scanning module, which position satisfies the first condition, is less than an area which corresponds to a possible position of the first scanning module, which position satisfies the second condition.

In an embodiment, the scanning result satisfying the first condition indicates that a distance between the first scanning module and the second scanning module is less than or equal to a first distance; the scanning result satisfying the second condition indicates that the distance between the first scanning module and the second scanning module is less than or equal to a second distance; wherein the first distance is less than the second distance; or
the scanning result satisfying the first condition indicates that a distance between the first scanning module and the second scanning module is less than or equal to a first distance; the scanning result satisfying the second condition indicates that the distance between the first scanning module and the second scanning module is greater than a third distance but less than or equal to a second distance; wherein the first distance is less than the second distance, the third distance is not greater than the first distance; or
the scanning result satisfying the first condition indicates that a strength of the scanning signal is greater than or equal to a first signal strength; the scanning result satisfying the second condition indicates that the strength of the scanning signal is greater than or equal to a second signal strength; wherein the first signal strength is greater than the second signal strength; or
the scanning result satisfying the first condition indicates that a strength of the scanning signal is greater than or equal to a first signal strength; the scanning result satisfying the second condition indicates that the strength of the scanning signal is less than a third signal strength but greater than or equal to a second signal strength; wherein the third signal strength is not less than the first signal strength, the first signal strength is greater than the second signal strength.

In an embodiment, the scanning result satisfying the first condition indicates that the first control apparatus is located inside a first area which is inside the operating room and capable of being scanned by the second scanning module;
the scanning result satisfying the second condition indicates that the first control apparatus is located inside a second area which is capable of being scanned by the second scanning module;
wherein the second area covers the operating room entirely, or the second area covers the first area and is greater than the first area.

In an embodiment, a communicative connection being established between the medical device and the control device, includes:
establishing, by the first control apparatus, a wireless connection between the control device and the first control apparatus, based on wireless connection information; and
performing, by the first control apparatus, relay communication between the medical device and the control device;
wherein the wireless connection information is transmitted from the second control apparatus to the first control apparatus, or is pre-stored by the first control apparatus.

In an embodiment, said communicative connection between the medical device and the control device being disconnected, includes:
disconnecting, by the first control apparatus, the wireless connection between the first control apparatus and the control device;
wherein the second control apparatus is configured to instruct the first control apparatus to disconnect the wireless connection, or the first control apparatus is configured to initiate to disconnect the wireless connection.

In an embodiment, a communicative connection being established between the medical device and the control device, includes:
transmitting, by the first control apparatus, wireless connection information to the medical device, so as to enable the medical device to establish a wireless connection with the control device based on the wireless connection information;
wherein the medical device is equipped with a wireless communication module, and the wireless connection information is transmitted from the second control apparatus to the first control apparatus, or the wireless connection information is pre-stored by the first control apparatus.

In an embodiment, said communicative connection between the medical device and the control device being disconnected, includes:
instructing the medical device, by the first control apparatus, to disconnect the wireless connection with the control device.

In an embodiment, transmitting the wireless connection information to the first control apparatus, includes: transmitting, by the second control apparatus, the wireless connection information to the first scanning module through the second scanning module.

In an embodiment, in order for the position of the first control apparatus to change synchronously with the position of the medical device, the first control apparatus is integrated to the medical device; or
in order for the position of the first control apparatus to change synchronously with the position of the medical device, the position of the first control apparatus and the position of the medical device are associated, when the first control apparatus is an independent apparatus in the control system.

In an embodiment, the second control apparatus is arranged on a ceiling of the operating room.

In an embodiment, the second control apparatus is an independent apparatus in the control system; or the second control apparatus is integrated to the control device; or the second control apparatus is integrated to a router of a wireless local area network where the control device is located, and the router is arranged inside the operating room.

In an embodiment, the first scanning module and the second scanning module each includes at least one of: a UWB module, a Wi-Fi module, an infrared module, and a Bluetooth module.

In an embodiment, the medical device includes at least one of: a laparoscope, a pneumoperitoneum machine, a flushing suction pump, and an energy platform.

A second aspect according to an embodiment of this disclosure provides a control system for an operating room, including: a control device for the operating room, a first control apparatus, and a second control apparatus;
the first control apparatus is in communicative connection with a medical device, wherein the first control apparatus includes a first scanning module, and a position of the first control apparatus changes synchronously with a position of the medical device;
the second control apparatus is arranged at a designated position of the operating room inside which room the control device is located, wherein the second control apparatus includes a second scanning module; the first scanning module is configured to receive a scanning signal which is transmitted by the second scanning module and/or the second scanning module is configured to receive a scanning signal which is transmitted by the first scanning module, so as to obtain a scanning result;
wherein:
   a communicative connection is established between the medical device and the control device, when the scanning result indicates that the first control apparatus is located inside a first area and no communicative connection has been established between the medical device and the control device; wherein the first area is an area, which is inside the operating room and capable of being scanned by the second control apparatus;
   a communicative connection is established between the medical device and the control device, when the scanning result indicates that the first control apparatus is located outside the first area, and no communicative connection has been established between the medical device and the control device, or a last communicative connection between the medical device and the control device has been disconnected and the first control apparatus or the medical device has not been moved out of the operating room since the last communicative connection was established between the medical device and the control device.

In an embodiment, the scanning result indicating that the first control apparatus is located inside a first area, includes: the scanning result indicates that a distance between the first scanning module and the second scanning module is less than or equal to a first distance; or the scanning result indicates that the first control apparatus is located inside the first area which is inside the operating room and capable of being scanned by the second control apparatus; or the scanning result indicates that a strength of the scanning signal is greater than or equal to a first signal strength;
the scanning result indicating that the first control apparatus is located outside the first area, includes: the scanning result indicates that the distance between the first scanning module and the second scanning module is greater than the first distance; or the scanning result indicates that the first control apparatus is located outside the first area which is inside the operating room and capable of being scanned by the second control apparatus; or the scanning result indicates that the strength of the scanning signal is less than the first signal strength.

In an embodiment, the first control apparatus or the medical device having not been moved out of the operating room since the last communicative connection was established between the medical device and the control device, includes:
the first control apparatus has not experienced a power outage, since the last communicative connection was established between the medical device and the control device;
the control system further includes an imaging device, which displays that the first control apparatus or the medical device has not been moved out of the operating room since the last communicative connection was established between the medical device and the control device; or
position information of the first control apparatus and/or of the medical device is compared with pre-stored map data of the operating room to determine that the first control apparatus or the medical device has not been moved out of the operating room since the last communicative connection was established between the medical device and the control device.

In an embodiment, when a communicative connection has been established between the medical device and the control device, said communicative connection is maintained between the medical device and the control device until said communicative connection is disconnected, regardless of whether the scanning result indicates that the first control apparatus is located inside the first area or not.

In an embodiment, when a communicative connection has been established between the medical device and the control device;
said communicative connection is maintained between the medical device and the control device, when the scanning result indicates that the first control apparatus is located inside the first area;
said communicative connection is maintained between the medical device and the control device, when the scanning result indicates that the first control apparatus is located outside the first area but inside a second area; and
said communicative connection between the medical device and the control device is disconnected, when the scanning result indicates that the first control apparatus is located outside the second area.

In an embodiment, when the scanning result indicates that the first control apparatus is located outside the first area but inside a second area; a communicative connection is established between the first control apparatus and the control device, when no communicative connection has been established between the medical device and the control device, or a last communicative connection between the medical device and the control device has been disconnected and the first control apparatus or the medical device has not been moved out of the operating room since the last communicative connection was established between the medical device and the control device.

In an embodiment, a communicative connection being established between the medical device and the control device, includes:
establishing, by the first control apparatus, a wireless connection between the control device and the first control apparatus, based on wireless connection information; and
performing, by the first control apparatus, relay communication between the medical device and the control device;
wherein the wireless connection information is transmitted from the second control apparatus to the first control apparatus, or is pre-stored by the first control apparatus.

In an embodiment, a communicative connection being established between the medical device and the control device, includes:
transmitting, by the first control apparatus, wireless connection information to the medical device, so as to enable the medical device to establish a wireless connection with the control device based on the wireless connection information;
wherein the medical device is equipped with a wireless communication module, and the wireless connection information is transmitted from the second control apparatus to the first control apparatus, or the wireless connection information is pre-stored by the first control apparatus.

In an embodiment, transmitting the wireless connection information to the first control apparatus, includes: transmitting, by the second control apparatus, the wireless connection information to the first scanning module through the second scanning module.

In an embodiment, in order for the position of the first control apparatus to change synchronously with the position of the medical device, the first control apparatus is integrated to the medical device; or
in order for the position of the first control apparatus to change synchronously with the position of the medical device, the position of the first control apparatus and the position of the medical device are associated, when the first control apparatus is an independent apparatus in the control system.

In an embodiment, the second control apparatus is arranged on a ceiling of the operating room.

In an embodiment, the second control apparatus is an independent apparatus in the control system; or the second control apparatus is integrated to the control device; or the second control apparatus is integrated to a router of a wireless local area network where the control device is located, and the router is arranged inside the operating room.

A third aspect according to an embodiment of this disclosure provides a control system for an operating room, including: a control device for the operating room, a first control apparatus, and a second control apparatus;
the first control apparatus is in communicative connection with a medical device, wherein the first control apparatus includes a first scanning module, and a position of the first control apparatus changes synchronously with a position of the medical device;
the second control apparatus is arranged at a designated position of the operating room inside which room the control device is located, wherein the second control apparatus includes a second scanning module; the first scanning module is configured to receive a scanning signal which is transmitted by the second scanning module and/or the second scanning module is configured to receive a scanning signal which is transmitted by the first scanning module, so as to obtain a scanning result;
wherein:
   when the scanning result indicates that the first control apparatus is located inside a first area and no communicative connection has been established between the medical device and the control device, a communicative connection is established between the medical device and the control device; wherein the first area is an area, which is inside the operating room and capable of being scanned by the second control apparatus;
   when the scanning result indicates that the first control apparatus is located outside the first area and no communicative connection has been established between the medical device and the control device, a communicative connection is not capable of being established between the medical device and the control device;
   when a communicative connection has been established between the medical device and the control device, said communicative connection is maintained between the medical device and the control device until said communicative connection is disconnected, regardless of whether the scanning result indicates that the first control apparatus is located inside the first area or not.

In an embodiment, the scanning result indicating that the first control apparatus is located inside a first area, includes: the scanning result indicates that a distance between the first scanning module and the second scanning module is less than or equal to a first distance; or the scanning result indicates that the first control apparatus is located inside the first area which is inside the operating room and capable of being scanned by the second control apparatus; or the scanning result indicates that a signal strength between the first scanning module and the second scanning module is greater than or equal to a first signal strength;
the scanning result indicating that the first control apparatus is located outside the first area, includes: the scanning result indicates that the distance between the first scanning module and the second scanning module is greater than the first distance; or the scanning result indicates that the first control apparatus is located outside the first area which is inside the operating room and capable of being scanned by the second control apparatus; or the scanning result indicates that the signal strength between the first scanning module and the second scanning module is less than the first signal strength.

In an embodiment, a communicative connection being established between the medical device and the control device, includes:
establishing, by the first control apparatus, a wireless connection between the control device and the first control apparatus, based on wireless connection information; and
performing, by the first control apparatus, relay communication between the medical device and the control device;
wherein the wireless connection information is transmitted from the second control apparatus to the first control apparatus, or is pre-stored by the first control apparatus.

In an embodiment, a communicative connection being established between the medical device and the control device, includes:
transmitting, by the first control apparatus, wireless connection information to the medical device, so as to enable the medical device to establish a wireless connection with the control device based on the wireless connection information;
wherein the medical device is equipped with a wireless communication module, and the wireless connection information is transmitted from the second control apparatus to the first control apparatus, or the wireless connection information is pre-stored by the first control apparatus.

In an embodiment, in order for the position of the first control apparatus to change synchronously with the position of the medical device, the first control apparatus is integrated to the medical device; or
in order for the position of the first control apparatus to change synchronously with the position of the medical device, the position of the first control apparatus and the position of the medical device are associated, when the first control apparatus is an independent apparatus in the control system.

In an embodiment, the second control apparatus is an independent apparatus in the control system; or the second control apparatus is integrated to the control device; or the second control apparatus is integrated to a router of a wireless local area network where the control device is located, and the router is arranged inside the operating room.

A fourth aspect according to an embodiment of this disclosure provides a control method for a control device for an operating room, including:
obtaining position information of a medical device;
when the position information satisfies a first condition and no communicative connection has been established between the medical device and the control device, establishing a communicative connection between the medical device and the control device;
when the position information does not satisfy the first condition and no communicative connection has been established between the medical device and the control device, maintaining no communicative connection between the medical device and the control device;
when the position information satisfies the first condition or a second condition and a communicative connection has been established between the medical device and the control device, maintaining said communicative connection between the medical device and the control device;
when the position information does not satisfy the first condition and the second condition and a communicative connection has been established between the medical device and the control device, disconnecting said communicative connection between the medical device and the control device;
wherein an area which corresponds to a possible position of the medical device, which position satisfies the first condition, is less than an area which corresponds to a possible position of the medical device, which position satisfies the second condition.

In an embodiment, the position information satisfying the first condition indicates that the medical device is located inside a first area, which is inside the operating room;
the scanning result satisfying the second condition indicates that the medical device is located inside a second area, which covers the operating room entirely, or covers the first area and is greater than the first area.

In an embodiment, the medical device includes at least one of: a laparoscope, a pneumoperitoneum machine, a flushing suction pump, and an energy platform.

A fifth aspect according to an embodiment of this disclosure provides a control method for a control device for an operating room, including:
obtaining position information of a medical device;
establishing a communicative connection between the medical device and the control device, when the position information satisfies a first condition and no communicative connection has been established between the medical device and the control device;
establishing a communicative connection between the medical device and the control device; when the position information does not satisfy the first condition, and no communicative connection has been established between the medical device and the control device, or a last communicative connection between the medical device and the control device has been disconnected and the medical device has not been moved out of the operating room since the last communicative connection was established between the medical device and the control device.

In an embodiment, the medical device having not been moved out of the operating room since the last communicative connection was established between the medical device and the control device, includes:
the medical device has not experienced a power outage, since the last communicative connection was established between the medical device and the control device;
an imaging device displays that the medical device has not been moved out of the operating room; or
position information of the medical device is compared with pre-stored map data of the operating room to determine that the medical device has not been moved out of the operating room since the last communicative connection was established between the medical device and the control device.

In an embodiment, the method further includes:
when a communicative connection has been established between the medical device and the control device, maintaining said communicative connection between the medical device and the control device until said communicative connection is disconnected, regardless of whether the position information indicates that the medical device is located inside a first area or not;
wherein the first area is an area which is inside the operating room.

In an embodiment, when a communicative connection has been established between the medical device and the control device, the method further includes:
maintaining said communicative connection between the medical device and the control device, when the position information satisfies the first condition or a second condition;
disconnecting said communicative connection between the medical device and the control device, when the position information does not satisfy the second condition;
wherein an area which corresponds to a possible position of the medical device, which position satisfies the first condition, is less than an area which corresponds to a possible position of the medical device, which position satisfies the second condition.

A sixth aspect according to an embodiment of this disclosure provides a control method for a control device for an operating room, including:
obtaining position information of a medical device;
when the position information satisfies a first condition and no communicative connection has been established between the medical device and the control device, establishing a communicative connection between the medical device and the control device,
when the position information does not satisfy the first condition and no communicative connection has been established between the medical device and the control device, being not capable of establishing a communicative connection between the medical device and the control device;
when a communicative connection has been established between the medical device and the control device, maintaining said communicative connection between the medical device and the control device until said communicative connection is disconnected, regardless of whether the position information satisfies the first condition or not.

A seventh aspect according to an embodiment of this disclosure provides a control device for an operating room, including a processor and a memory, wherein the memory is configured to store a computer program which is executed by the processor, wherein the above control method is capable of being implemented when the computer program is executed by the processor.

An eighth aspect according to an embodiment of this disclosure provides a method for controlling connection of an operating room, which method is applicable to a control system for the operating room, wherein the control system includes a control device for the operating room, a first control apparatus, and a second control apparatus; wherein the first control apparatus is in communicative connection with a medical device, and a position of the first control apparatus changes synchronously with a position of the medical device; the second control apparatus is arranged at a designated position of the operating room inside which room the control device is located; wherein the method includes:
obtaining a scanning result between the first control apparatus and the second control apparatus;
when the scanning result satisfies a first condition and no communicative connection has been established between the medical device and the control device, establishing a communicative connection between the medical device and the control device;
when the scanning result does not satisfy the first condition and no communicative connection has been established between the medical device and the control device, maintaining no communicative connection between the medical device and the control device;
when the scanning result satisfies the first condition or satisfies a second condition and a communicative connection has been established between the medical device and the control device, maintaining said communicative connection between the medical device and the control device;
when the scanning result does not satisfy the first condition and the second condition and a communicative connection has been established between the medical device and the control device, disconnecting said communicative connection between the medical device and the control device;
wherein, when the scanning result indicates a distance, a maximum distance which satisfies the first condition, is less than a maximum distance which satisfies the second condition; or
when the scanning result indicates a strength of a scanning signal, a minimum signal strength which satisfies the first condition is greater than a minimum signal strength which satisfies the second condition; or
when the scanning result indicates a position of the first control apparatus, an area which corresponds to a possible position of the first control apparatus, which position satisfies the first condition, is less than an area which corresponds to a possible position of the first control apparatus, which position satisfies the second condition.

In an embodiment, the scanning result satisfying the first condition indicates that a distance between the first control apparatus and the second control apparatus is less than or equal to a first distance; the scanning result satisfying the second condition indicates that the distance between the first control apparatus and the second control apparatus is less than or equal to a second distance; wherein the first distance is less than the second distance; or
the scanning result satisfying the first condition indicates that a distance between the first control apparatus and the second control apparatus is less than or equal to a first distance; the scanning result satisfying the second condition indicates that the distance between the first control apparatus and the second control apparatus is greater than a third distance but less than or equal to a second distance; wherein the first distance is less than the second distance, the third distance is not greater than the first distance; or
the scanning result satisfying the first condition indicates that a strength of the scanning signal is greater than or equal to a first signal strength; the scanning result satisfying the second condition indicates that the strength of the scanning signal is greater than or equal to a second signal strength; wherein the first signal strength is greater than the second signal strength; or
the scanning result satisfying the first condition indicates that a strength of the scanning signal is greater than or equal to a first signal strength; the scanning result satisfying the second condition indicates that the strength of the scanning signal is less than a third signal strength but greater than or equal to a second signal strength; wherein the third signal strength is not less than the first signal strength, the first signal strength is greater than the second signal strength.

In an embodiment, the scanning result satisfying the first condition indicates that the first control apparatus is located inside a first area which is inside the operating room and capable of being scanned by the second control apparatus;
the scanning result satisfying the second condition indicates that the first control apparatus is located inside a second area which is capable of being scanned by the second control apparatus; wherein the second area covers the operating room entirely, or the second area covers the first area and is greater than the first area.

In an embodiment, establishing a communicative connection between the medical device and the control device, includes:
establishing, by the first control apparatus, a wireless connection between the control device and the first control apparatus, based on wireless connection information; and
performing, by the first control apparatus, relay communication between the medical device and the control device;
wherein the wireless connection information is transmitted from the second control apparatus to the first control apparatus, or is pre-stored by the first control apparatus.

In an embodiment, disconnecting said communicative connection between the medical device and the control device, includes:
disconnecting, by the first control apparatus, the wireless connection between the first control apparatus and the control device;
wherein the second control apparatus is configured to instruct the first control apparatus to disconnect the wireless connection, or the first control apparatus is configured to initiate to disconnect the wireless connection.

In an embodiment, establishing a communicative connection between the medical device and the control device, includes:
transmitting, by the first control apparatus, wireless connection information to the medical device, so as to enable the medical device to establish a wireless connection with the control device based on the wireless connection information;
wherein the medical device is equipped with a wireless communication module, and the wireless connection information is transmitted from the second control apparatus to the first control apparatus, or the wireless connection information is pre-stored by the first control apparatus.

In an embodiment, disconnecting said communicative connection between the medical device and the control device, includes:
instructing the medical device, by the first control apparatus, to disconnect the wireless connection with the control device.

A ninth aspect according to an embodiment of this disclosure provides a method for controlling connection of an operating room, which method is applicable to a control system for the operating room, wherein the control system includes a control device for the operating room, a first control apparatus, and a second control apparatus; wherein the first control apparatus is in communicative connection with a medical device, and a position of the first control apparatus changes synchronously with a position of the medical device; the second control apparatus is arranged at a designated position of the operating room inside which room the control device is located; wherein the method includes:
obtaining a scanning result between the first control apparatus and the second control apparatus;
establishing a communicative connection between the medical device and the control device, when the scanning result indicates that the first control apparatus is located inside a first area and no communicative connection has been established between the medical device and the control device; wherein the first area is an area, which is inside the operating room and capable of being scanned by the second control apparatus;
establishing a communicative connection between the medical device and the control device, when the scanning result indicates that the first control apparatus is located outside the first area, and no communicative connection has been established between the medical device and the control device, or a last communicative connection between the medical device and the control device has been disconnected and the first control apparatus or the medical device has not been moved out of the operating room since the last communicative connection was established between the medical device and the control device.

In an embodiment, the scanning result indicating that the first control apparatus is located inside a first area, includes: the scanning result indicates that a distance between the first control apparatus and the second control apparatus is less than or equal to a first distance; or the scanning result indicates that the first control apparatus is located inside the first area which is inside the operating room and capable of being scanned by the second control apparatus; or the scanning result indicates that a strength of a scanning signal is greater than or equal to a first signal strength;
the scanning result indicating that the first control apparatus is located outside the first area, includes: the scanning result indicates that the distance between the first control apparatus and the second control apparatus is greater than the first distance; or the scanning result indicates that the first control apparatus is located outside the first area which is inside the operating room and capable of being scanned by the second control apparatus; or the scanning result indicates that the strength of the scanning signal is less than the first signal strength.

In an embodiment, the first control apparatus or the medical device having not been moved out of the operating room since the last communicative connection was established between the medical device and the control device, includes:
the first control apparatus has not experienced a power outage, since the last communicative connection was established between the medical device and the control device;
the control system further includes an imaging device, which displays that the first control apparatus or the medical device has not been moved out of the operating room since the last communicative connection was established between the medical device and the control device; or
position information of the first control apparatus and/or of the medical device is compared with pre-stored map data of the operating room to determine that the first control apparatus or the medical device has not been moved out of the operating room since the last communicative connection was established between the medical device and the control device.

In an embodiment, when a communicative connection has been established between the medical device and the control device, the method further includes:
maintaining said communicative connection between the medical device and the control device until said communicative connection is disconnected, regardless of whether the scanning result indicates that the first control apparatus is located inside the first area or not.

In an embodiment, when a communicative connection has been established between the medical device and the control device, the method further includes:
maintaining said communicative connection between the medical device and the control device, when the scanning result indicates that the first control apparatus is located inside the first area;
maintaining said communicative connection between the medical device and the control device, when the scanning result indicates that the first control apparatus is located outside the first area but inside a second area; and
disconnecting said communicative connection between the medical device and the control device, when the scanning result indicates that the first control apparatus is located outside the second area.

In an embodiment, when the scanning result indicates that the first control apparatus is located outside the first area but inside a second area, the method further includes:
establishing a communicative connection between the first control apparatus and the control device, when no communicative connection has been established between the medical device and the control device, or a last communicative connection between the medical device and the control device has been disconnected and the first control apparatus or the medical device has not been moved out of the operating room since the last communicative connection was established between the medical device and the control device.

A tenth aspect according to an embodiment of this disclosure provides a method for controlling connection of an operating room, which method is applicable to a control system for the operating room, wherein the control system includes a control device for the operating room, a first control apparatus, and a second control apparatus; wherein the first control apparatus is in communicative connection with a medical device, and a position of the first control apparatus changes synchronously with a position of the medical device; the second control apparatus is arranged at a designated position of the operating room inside which room the control device is located; wherein the method includes:
obtaining a scanning result between the first control apparatus and the second control apparatus;
when the scanning result indicates that the first control apparatus is located inside a first area and no communicative connection has been established between the medical device and the control device, establishing a communicative connection between the medical device and the control device; wherein the first area is an area, which is inside the operating room and capable of being scanned by the second control apparatus;
when the scanning result indicates that the first control apparatus is located outside the first area and no communicative connection has been established between the medical device and the control device, being not capable of establishing a communicative connection between the medical device and the control device;
when a communicative connection has been established between the medical device and the control device, maintaining said communicative connection between the medical device and the control device until said communicative connection is disconnected, regardless of whether the scanning result indicates that the first control apparatus is located inside the first area or not.

In an embodiment, the scanning result indicating that the first control apparatus is located inside a first area, includes: the scanning result indicates that a distance between the first control apparatus and the second control apparatus is less than or equal to a first distance; or the scanning result indicates that the first control apparatus is located inside the first area which is inside the operating room and capable of being scanned by the second control apparatus; or the scanning result indicates that a strength of a scanning signal between the first control apparatus and the second control apparatus is greater than or equal to a first signal strength;
the scanning result indicating that the first control apparatus is located outside the first area, includes: the scanning result indicates that the distance between the first control apparatus and the second control apparatus is greater than the first distance; or the scanning result indicates that the first control apparatus is located outside the first area which is inside the operating room and capable of being scanned by the second control apparatus; or the scanning result indicates that the strength of the scanning signal between the first control apparatus and the second control apparatus is less than the first signal strength.

An eleventh aspect according to an embodiment of this disclosure provides a control apparatus, which is arranged at a designated position of an operating room;
the control apparatus includes a first scanning module, a first processor, a first memory, wherein the first memory is configured to store a computer program which is executed by the first processor, wherein any one method mentioned above is capable of being implemented when the computer program is executed by the first processor.

A twelfth aspect according to an embodiment of this disclosure a control apparatus, which is applicable to a control system for an operating room, wherein the control apparatus is in communicative connection with a medical device, and a position of the control apparatus changes synchronously with a position of the medical device;
the control apparatus includes a second scanning module, a second processor, and a second memory, wherein the second memory is configured to store a computer program which is executed by the second processor, wherein any one method mentioned above is capable of being implemented when the computer program is executed by the second processor.

A thirteenth aspect according to an embodiment of this disclosure provides a control device for an operating room, including a third processor and a third memory, wherein the third memory is configured to store a computer program which is executed by the third processor, wherein any one method mentioned above is capable of being implemented when the computer program is executed by the third processor.

The control system for an operating room, the method for controlling connection of an operating room, the control apparatus, and the control device for an operating room discussed in embodiments of this disclosure, are capable of establishing, disconnecting, or maintaining a communicative connection between the medical device and the control device for the operating room, based on a scanning result which indicates a distance or a signal strength, without requiring manual operation by a user. In addition, by setting at least partially different conditions for establishing, maintaining, and disconnecting a connection, and by setting stricter condition for establishing a connection than that for maintaining/disconnecting a connection, an inaccurate connection with another operating room, as well as a problem, such as an unstable communicative connection, which is caused by signal obstruction or movement after connection, can be avoided.

When the medical device is disconnected from the control device, the embodiments of this disclosure can also achieve a reconnection by determining whether the medical device has been moved out of the operating room since a last connection with the control device has been established, thus solving the problem of accidental disconnection.

### BRIEF DESCRIPTION OF THE DRAWINGS

By providing a more detailed description of embodiments of this disclosure in conjunction with accompanying drawings, the above and other objectives, features, and advantages of this disclosure become more apparent. The accompanying drawings are used to provide further understanding of the embodiments of this disclosure and form a part of the description, and used together with the embodiments to explain this disclosure and do not constitute limitations on this disclosure. In the accompanying drawings, same reference numbers usually indicate same components or steps.
FIG. 1 is a block diagram of a control system for an operating room according to an embodiment of this disclosure.
FIG. 2 is a diagram of a layout position of a control system for an operating room inside the operating room according to one embodiment of this disclosure.
FIG. 3 is a diagram of a layout position of a control system for an operating room inside the operating room according to another embodiment of this disclosure.
FIG. 4 is a flowchart of a method for controlling connection of an operating room according to an embodiment of this disclosure.
FIG. 5 is a flowchart of a method for controlling connection of an operating room according to an embodiment of this disclosure.
FIG. 6 is a flowchart of a method for controlling connection of an operating room according to an embodiment of this disclosure.
FIG. 7 is a block diagram of a control apparatus according to an embodiment of this disclosure.
Figure 8 is a block diagram of a control apparatus according to another embodiment of this disclosure.
FIG. 9 is a block diagram of a control device for an operating room according to an embodiment of this disclosure.
FIG. 10 is a flowchart of a control method for a control device for an operating room according to an embodiment of this disclosure.
FIG. 11 is a flowchart of a control method for a control device for an operating room according to an embodiment of this disclosure.
FIG. 12 is a flowchart of a control method for a control device for an operating room according to an embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments obtained by those skilled in the art without creative work, fall within the protection scope of this disclosure.

In the following description, a large number of specific details are provided to provide a more thorough understanding of this disclosure. However, it is evident to those skilled in the art that this disclosure can be implemented without requiring one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "includes", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

At present, wired or wireless communication between a medical device and a control device for an operating room requires manual operation by user(s). For wireless communication, connection and disconnection are based on a communication distance/signal strength of the wireless communication itself. To automatically establish a communicative connection with the control device when the medical device moves to an operating room, a usual method is to establish a communicative connection between the medical device and the control device for the operating room, when a distance measured is less than a preset distance. When the distance measured between the medical device and the control device is greater than the preset distance, the communicative connection between the medical device and the control device is disconnected. When the medical device is obstructed, a measurement result for the distance may be relatively greater than an actual value, and the communicative connection may be disconnected, thereby affecting the stability of the communicative connection.

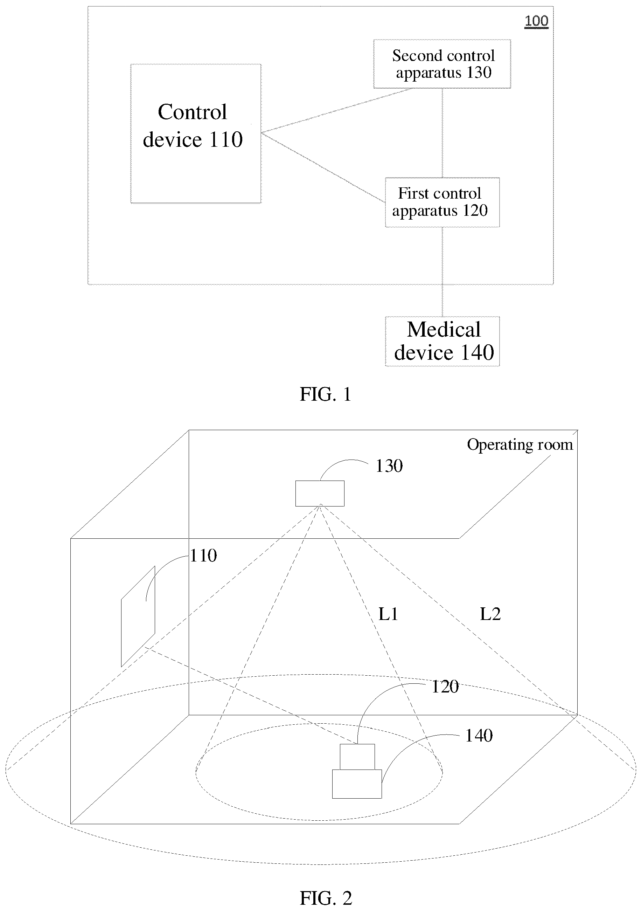

An embodiment of this disclosure provides a control system for an operating room, as shown in FIG. 1. The control system 100 for an operating room of the embodiment of this disclosure includes a control device 110 for the operating room, a first control apparatus 120, and a second control apparatus 130. The first control apparatus 120 is in communicative connection with a medical device 140. The first control apparatus 120 includes a first scanning module. A position of the first control apparatus 120 changes synchronously with a position of the medical device 140. The second control apparatus 130 is arranged at a designated position of the operating room inside which room the control device 110 is located. The second control apparatus 130 includes a second scanning module. The first scanning module is configured to receive a scanning signal which is transmitted by the second scanning module and/or the second scanning module is configured to receive a scanning signal which is transmitted by the first scanning module, so as to obtain a scanning result. The scanning result is capable of indicating a distance, a signal strength, or a position of the first control apparatus 120 inside the operating room. The control system 100 for an operating room is capable of establishing, disconnecting, or maintaining a communicative connection between the medical device 140 and the control device 110 for the operating room, based on analysis and determination on the scanning result.

When a communicative connection is established between the medical device 140 and control device 110, the control device 110 is capable of controlling the medical device 140. Specifically, based on the communicative connection between the control device 110 and the medical device 140, instruction(s) or data can be transmitted between the control device 110 and the medical device 140, including but not limited to that the control device 110 issues instruction(s) to the medical device 140, the medical device 140 returns response(s) to the control device 110, or the medical device 140 reports data to the control device 110. In the embodiments of this disclosure, the control device 110 is capable of directly communicating with the medical device 140, or indirectly communicating with the medical device 140 via the first control apparatus 120.

Furthermore, at least one of a Wi-Fi module, a Bluetooth module, a controller area network (CAN) interface, and an RS485 interface can be configured in the first control apparatus 120 and the second control apparatus 130, so as to support a wired communicative connection with a medical device through the CAN interface and/or RS485 interface, or wireless LAN communication with a medical device through Wi-Fi, or short-range wireless communication with a medical device through Bluetooth.

The first control apparatus 120 can exist as an independent device in the control system 100 through independent packaging, or can be integrated to the medical device 140, for example, integrated to a medical device 140 with Wi-Fi function. The position of the first control apparatus 120 changes synchronously with a position of at least one medical device 140, wherein the medical device includes a movable medical device, such as an endoscope, a pneumoperitoneum machine, a flushing suction pump, an energy platform, etc., which is used inside the operating room.

In order for the position of the first control apparatus 120 to change synchronously with the position of the medical device 140, the first control apparatus 120 can be integrated to said medical device. When the first control apparatus 120 is an independent device in the control system 100, the position of the first control apparatus 120 and the position of said medical device 140 can be associated, so as to synchronously change the position of the first control apparatus 120 with the position of said medical device 140. Specifically, the position of the first control apparatus 120 changing synchronously with the position of the medical device, can be understood as that the first control apparatus 120 and the medical device 140 are located in a same area regardless of how they move, or as that a distance between the first control apparatus 120 and the medical device 140 is always less than a preset threshold, or as that a displacement of the first control apparatus 120 in a certain direction causes an associated displacement of the medical device 140 in an associated direction, or as that a displacement of the medical device 140 in a certain direction causes an associated displacement of the first control apparatus 120 in an associated direction. For example, the first control apparatus 120 can be externally mounted on the medical device 140 to achieve a synchronized position change, or both the first control apparatus 120 and the medical device 140 can be placed on a vehicle to achieve a synchronized position change.

The first control apparatus 120 is capable of establishing a communicative connection with the control device 110 and at least one medical device 140 through wired or wireless means. In a specific example, the first control apparatus 120 includes a first wired communication module and a first wireless communication module, and establishes a communicative connection with the control device 110 through the first wireless communication module, and establishes a communicative connection with at least one medical device 140 through the first wired communication module.

For example, the first wireless communication module includes at least one of: a Wi-Fi module and a Bluetooth module; the first wired communication module includes at least one of: a CAN interface and an RS485 interface.

The second control apparatus 130 is arranged at a designated position of an operating room inside which room the control device 110 is located, that is, the deployment position of the second control apparatus 130 inside the operating room is fixed. As an implementation method, the second control apparatus 130 can be arranged on a ceiling of the operating room, and further, the second control apparatus 130 can be arranged at a center position of the ceiling of the operating room. When the second control apparatus 130 is arranged on the ceiling of the operating room, signals transmitted by the second control apparatus 130 can cover the entire operating room, and signals from the first control apparatus 120 in various areas of the operating room can also be received by the second control apparatus 130, thus avoiding a problem of signal obstruction by numerous other devices inside the operating room.

In practical applications, the second control apparatus 130 can exist as an independent device in the control system 100 through independent packaging, or can be integrated to the control device 110. Alternatively, if a router is set up in a wireless local area network where the control device 110 is located, the second control apparatus 130 can also be integrated to the router. Usually, wireless routers are arranged in wireless LANs. For signal quality considerations, wireless routers are also arranged inside unobstructed areas around the operating room or areas with good communication link quality. Therefore, integrating the second control apparatus 130 into the router can ensure signal transmission and reception quality of the second control apparatus 130. Optionally, a device form of the second control apparatus 130 can also be a terminal device, such as a mobile phone, a watch, a positioning device, etc.

For example, the second control apparatus 130 may include a second wired communication module and a second wireless communication module, so as to establish a communicative connection with the control device 110 through wired or wireless means, and establish a communicative connection with one or more medical devices 140 through wired or wireless means. Due to the fixed position of the second control apparatus 130, the medical device 140, that establishes a communicative connection with the second control apparatus 130, may be a medical device with a relatively fixed position inside the operating room.

In a specific example, the control device 110, the first control apparatus 120, and the second control apparatus 130 are connected with a same wireless LAN through one or more routing devices. Wherein, the first control apparatus 120 is in a communicative connection with a pneumoperitoneum machine, an energy platform, a suction flushing pump, and an endoscope, and the second control apparatus 130 is in a communicative connection with an operating table and an operating lamp. It can be understood that the operating table and the operating lamp are usually used in a fixed operating room and are not shared with other operating rooms. Therefore, this type of medical device, that does not move often/will not move, can establish a communicative connection with the second control apparatus 130 located at a fixed position inside the operating room. Devices, such as a pneumoperitoneum machine, an energy platform, a suction flushing pump, and an endoscope, may be moved to different operating rooms for use. Therefore, these movable/constantly moving medical devices can establish a communicative connection with the first control apparatus 120, and their positions change synchronously.

Wherein, the first control apparatus 120 or the second control apparatus 130 can serve as a relay device for relay communication between the control device 110 and the medical device 140. When the medical device 140 is equipped with a wireless communication module, the medical device 140 can also be connected in a wireless LAN where the control device 110 is located, and establish a communicative connection with the control device 110.

Generally speaking, the control device 110 only needs to establish a communicative connection with a medical device 140 which is currently used in the operating room, so as to control the medical device 140 or obtain data from the medical device 140. Since the position of the first control apparatus 120 changes synchronously with the position of the medical device 140, it is possible to determine the position of the medical device 140 by determining the position of the first control apparatus 120, and then determine whether to establish a communicative connection between the medical device 140 and the control device 110. Due to the relatively fixed position of the second control apparatus 120 inside the operating room, a relative position between the first control apparatus 120 and the second control apparatus 130 can be determined, so as to determine the position of the medical device 140 inside the operating room.

As described above, the first control apparatus 120 includes a first scanning module, and the second control apparatus 130 includes a second scanning module. The first scanning module can transmit a scanning signal and the second scanning module can receive the scanning signal to obtain a scanning result; or the second scanning module can transmit a scanning signal and the first scanning module can receive the scanning signal to obtain a scanning result. The scanning result can indicate a distance, a signal strength, or a position of the first scanning module (i.e., the position of the first control apparatus 120), such that a communicative connection between the medical device 140 and the control device 110 can be established, disconnected, or maintained based on the scanning result.

Wherein, the first scanning module can be a UWB (ultra-wideband) module, a Bluetooth module, a Wi-Fi module, or an infrared module, and the second scanning module is of the same type as the first scanning module. The first scanning module and the second scanning module can scan each other based on a mutually supported communication method, so as to obtain the scanning result. For example, the first scanning module and the second scanning module can both be UWB modules, and they scan each other based on UWB technology. UWB technology is a wireless carrier communication technology that has advantages of low system complexity, low spectral density of transmission signal power, insensitivity to channel fading, low interception capability, and high positioning accuracy. Therefore, it is suitable for high-speed wireless access in dense multipath environments, such as operating rooms.

When the first scanning module and the second scanning module are UWB modules, the first scanning module can transmit a UWB signal, and the second scanning module can receive the UWB signal. Based on time difference between transmission and reception of the UWB signal, a scanning result which indicates a distance can be obtained. Alternatively, the second scanning module can transmit a UWB signal and the first scanning module can receive the UWB signal to obtain the scanning result. The UWB module can also utilize an ultra-wideband characteristic of signal for high-precision positioning, so as to obtain a scanning result which indicates the position of the first scanning module. When the first scanning module and the second scanning module are Bluetooth modules, infrared modules, or Wi-Fi modules, the scanning result which indicates the distance can be obtained based on flight time, or the scanning result which indicates a signal strength can be obtained by performing scanning between the first scanning module and the second scanning module.

In one embodiment of this disclosure, a strategy for controlling a communicative connection between the medical device 140 and the control device 110 based on the scanning result, includes:
establishing a communicative connection between the medical device 140 and the control device 110, when the scanning result satisfies a first condition and no communicative connection has been established between the medical device 140 and the control device 110;
otherwise, maintaining no communicative connection between the medical device 140 and the control device 110, when the scanning result does not satisfy the first condition, and no communicative connection has been established between the medical device 140 and the control device 110, that is, no communicative connection between the medical device 140 and the control device 110 is established at this time;
when the scanning result satisfies the first condition or a second condition and a communicative connection has been established between the medical device 140 and the control device 110, maintaining said communicative connection between the medical device 140 and the control device 110;
when the scanning result does not satisfy the first condition and the second condition and a communicative connection has been established between the medical device 140 and the control device 110, disconnecting said communicative connection between the medical device 140 and the control device 110.

Wherein, the scanning result satisfying the first condition indicates that the first control apparatus 120 enters the operating room, or enters a certain range inside the operating room, or enters a certain range which is centered around the operating room. For example, when the medical device 140, along with the first control apparatus 120, is moved from another operating room to a current operating room, the scanning result satisfies the first condition when the medical device 140 and the first control apparatus 120 enter the current operating room, and then a communicative connection is established between the medical device 140 and the control device 110.

For example, the scanning result satisfying the first condition indicates that a distance between the first scanning module and the second scanning module is less than or equal to a first distance. Alternatively, the scanning result satisfying the first condition indicates that a strength of the scanning signal is greater than or equal to a first signal strength. That is because the closer the distance, the higher the strength of the scanning signal. In addition, when the distance between the first scanning module and the second scanning module cannot be directly measured, the distance between the first scanning module and the second scanning module can be indirectly reflected based on the strength of the scanning signal obtained by both scanning modules.

When a communicative connection has been established between the medical device 140 and the control device, the first scanning module and/or the second scanning module can continue scanning. As long as the scanning result satisfies the first condition, the communicative connection between the medical device and the control device is maintained. Moreover, if the scanning result does not satisfy the first condition but satisfies the second condition, the communicative connection between the medical device and the control device is also maintained.

For example, the scanning result satisfying the first condition indicates that the first control apparatus 120 is located in a first area which is inside the operating room and capable of being scanned by the second scanning module; the scanning result satisfying the second condition indicates that the first control apparatus 120 is located inside a second area which is capable of being scanned by the second scanning module. Referring to FIG. 2, the second control apparatus 130 is arranged behind the ceiling. A possible position of the first control apparatus 120 which position satisfies the first condition, forms a first area on a ground plane of the operating room, which area is less than or equal to a first distance L1. A projection of the second control apparatus 130 is located at the center of this area. The first area can be an internal area of the operating room, for example, a circular area which is formed by the projection on the ground plane of the operating room, and a boundary of this circular area does not exceed a boundary of the operating room. The possible position of the first control apparatus 120, which position satisfies the second condition, forms a second area on the ground plane of the operating room, which area is less than or equal to the second distance L2. This second area covers more circular areas of the operating room than the first area. One possible scenario is that the second area covers an entire floor area of the operating room. Alternatively, another possible scenario is that the second area may only cover a portion of the operating room, and the second area may cover the first area and be greater than the first area. For example, the second area may not cover corners of the operating room.

Specifically, when the scanning result indicates a distance, a maximum distance which satisfies the first condition, is less than a maximum distance which satisfies the second condition. For example, the scanning result satisfying the first condition indicates that the distance between the first scanning module and the second scanning module is less than or equal to a first distance L1; and the scanning result satisfying the second condition indicates that the distance between the first scanning module and the second scanning module is less than or equal to a second distance L2, wherein L1 <L2. When the distance between the first scanning module and the second scanning module is greater than L1, but less than or equal to L2, the communicative connection between the medical device and the control device is still maintained, rather than disconnected due to the distance being greater than L1. When the first control apparatus 120 is obstructed, a measurement result of the distance is increased, and in this embodiment of this disclosure, the communicative connection between the medical device and the control device is not disconnected but maintained within a distance interval from L1 to L2, which improves the stability of the communicative connection.

In another embodiment, the scanning result satisfying the second condition indicates that the distance between the first scanning module and the second scanning module is greater than a third distance L3 but less than or equal to a second distance L2; wherein the second distance L2 is greater than the first distance L1, the third distance L3 is not greater than the first distance L1. As long as the distance between the first scanning module and the second scanning module is less than or equal to L2, the communicative connection between the medical device 140 and the control device 110 is not disconnected.

When the scanning result indicates a strength of the scanning signal, a minimum signal strength which satisfies the first condition is greater than a minimum signal strength which satisfies the second condition. For example, the scanning result satisfying the first condition indicates that a strength of the scanning signal is greater than or equal to a first signal strength A1; and the scanning result satisfying the second condition indicates that the strength of the scanning signal is greater than or equal to a second signal strength A2, wherein A1>A2. When the strength of the scanning signal is less than A1, but greater than or equal to A2, the communicative connection between the medical device and the control device is still maintained, and the communicative connection will not be disconnected due to the strength of the scanning signal being less than A1. When the first control apparatus 120 is obstructed, a measurement result of the strength of the scanning signal is decreased, and in this embodiment of this disclosure, the communicative connection between the medical device and the control device is not disconnected but maintained within a strength range of signal from A1 to A2, which improves the stability of the communicative connection.

In another embodiment, the scanning result satisfying the first condition indicates that a strength of the scanning signal is greater than or equal to a first signal strength A1; the scanning result satisfying the second condition indicates that the strength of the scanning signal is less than a third signal strength A3 but greater than or equal to a second signal strength A2; wherein the third signal strength A3 is not less than the first signal strength A1, the first signal strength A1 is greater than the second signal strength A2. As long as the signal strength is greater than or equal to the second signal strength A2, the communicative connection between the medical device and the control device is not disconnected.

Alternatively, when the scanning result indicates a position of the first scanning module, an area which corresponds to a possible position of the first scanning module, which position satisfies the first condition, is less than an area which corresponds to a possible position of the first scanning module, which position satisfies the second condition. For example, if satisfying the first condition requests the first scanning module to be located in an area S 1, and satisfying the second condition requests the first scanning module to be located in an area S2, the area S1 is inside the area S2 and has a surface area which is less than that of the area S2.

When the scanning result does not satisfy the first condition and the second condition, and a communicative connection has been established between the medical device 140 and the control device 110, said communicative connection between the medical device 140 and the control device 110 is disconnected. When the scanning result does not satisfy the second condition, it may indicate that the medical device 140 has moved out of a certain range around the operating room, rather than the distance measurement result is increased or the signal strength is decreased due to the obstruction of the first control apparatus. Therefore, the control device 110 no longer needs to control the medical device 140. At this time, the communicative connection between the medical device 140 and the control device 110 can be disconnected to facilitate subsequent establishment of communicative connection(s) between the medical device 140 and other control device(s).

According to the above strategy of communicative connection, it is possible to automatically establish a wireless connection between the medical device 140 and the control device 110 without requiring manual operation of user. When the medical device 140 is moved into the operating room, the medical device 140 can automatically be in communicative connection with the control device, and when it is moved out of the operating room, the communicative connection can be automatically disconnected. The condition for establishing a communicative connection is limited to the operating room, which can prevent the medical device 140 from mistakenly connecting with other control devices in other operating rooms. The second condition for maintaining communicative connection covers more areas of the operating room compared to the first condition, which can avoid the problem of disconnection due to obstruction, movement to the corner of the operating room, or slight departure from the first area, and can improve the stability of communicative connection. Setting the second condition for disconnection can achieve an automatic disconnection of the medical device 140 after leaving the operating room. In summary, the above strategy of communicative connection can achieve seamless connection and disconnection between the medical device 140 and the control device 110, as well as avoid the occurrence of accidental disconnection.

In another embodiment of this disclosure, a strategy for controlling communicative connection between the medical device 140 and the control device 110 based on the scanning result, includes:
establishing a communicative connection between the medical device 140 and the control device 110; when the scanning result indicates that the first control apparatus 120 is located inside a first area, and no communicative connection has been established between the medical device 140 and the control device 110; wherein the first area is an area which is inside the operating room and capable of being scanned by the second control apparatus 130;
establishing a communicative connection between the medical device 140 and the control device 110; when the scanning result indicates that the first control apparatus 120 is located outside the first area, and no communicative connection has been established between the medical device 140 and the control device 110, or a last communicative connection between the medical device 140 and the control device 110 has been disconnected and the first control apparatus 120 or the medical device 140 has not been moved out of the operating room since the last communicative connection was established between the medical device and the control device.

Wherein, the scanning result indicates a signal strength, a distance, or a position of the first control apparatus. The scanning result indicating that the first control apparatus is located inside a first area, includes: the scanning result indicates that a distance between the first scanning module and the second scanning module is less than or equal to a first distance; or the scanning result indicates that the first control apparatus is located inside the first area which is inside the operating room and capable of being scanned by the second scanning module; or the scanning result indicates that a strength of a scanning signal is greater than or equal to a first signal strength. When the scanning result satisfies any of the above conditions, the first control apparatus 120 is located inside the first area.

On the contrary, the scanning result indicating that the first control apparatus is located outside the first area, includes: the scanning result indicates that the distance between the first scanning module and the second scanning module is greater than the first distance; or the scanning result indicates that the first control apparatus is located outside the first area which is inside the operating room and capable of being scanned by the second scanning module; or the scanning result indicates that the strength of the scanning signal is less than the first signal strength. When the scanning result satisfies any of the above conditions, the first control apparatus 120 is located outside the first area.

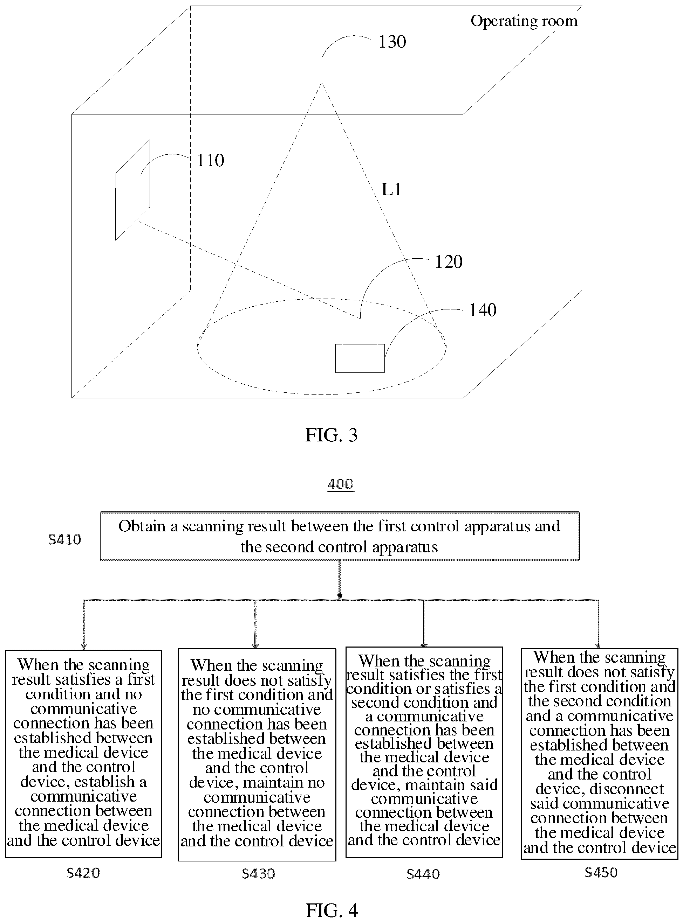

For example, referring to FIG. 3, when the scanning result indicates that the distance between the first scanning module and the second scanning module is less than or equal to a first distance L1, then the first area is a circular area which is located inside the operating room, and a projection of the second control apparatus 130 is located at a centre of the circular area.

According to the above strategy, if no communicative connection has originally been established between the medical device 140 and the control device 110; a communicative connection is established between the medical device 140 and the control device 110, when the scanning result indicates that the first control apparatus 120 enters into the first area from outside. After establishing a communicative connection between the medical device 140 and the control device 110, when the scanning result indicates that the first control apparatus 120 is located inside the first area, said communicative connection between the medical device 140 and the control device 110 is always maintained. For example, when a medical device 140, that has not established a communicative connection with the control device 110, enters the first area of the operating room together with the first control apparatus 120, a communicative connection is established between the medical device 140 and the control device 110, so as to facilitate control of the medical device 140 by the control device 110.

After establishing a communicative connection between the medical device 140 and the control device 110, when the scanning result indicates that the first control apparatus 120 has left the first area, but the first control apparatus 120 or the medical device 140 is determined to have not been moved out of the operating room, then the communicative connection between the medical device 140 and the control device 110 can be reestablished, when the communicative connection between the medical device 140 and the control device 110 is disconnected. For example, if the communicative connection is accidentally disconnected due to obstruction of the medical device 140 or abnormal wireless signal quality, as long as the first control apparatus 120 or the medical device 140 is always located inside the operating room, the communicative connection between the medical device 140 and the control device 110 will be automatically reestablished until the communicative connection has been successfully established, thereby avoiding the problem of unstable communicative connection.

Furthermore, under a condition without a power outage, as long as the first control apparatus 120 or the medical device 140 is determined to have not been moved out of the operating room, the communicative connection between the medical device 140 and the control device 110 is reestablished. Alternatively, in order to reestablish the communicative connection between the medical device 140 and the control device 110, it is necessary to ensure that the scanning result indicates that the first control apparatus 120 is located inside a second area which is greater than the first area.

If the first control apparatus 120 does not experience a power outage after a last communicative connection between the medical device 140 and the control device 110 is established, the first control apparatus 120 or the medical device 140 is determined to have not been moved out of the operating room since the last communicative connection was established between the medical device and the control device. Specifically, since the first control apparatus 120 and the medical device 140 are usually powered by a trolley, the first control apparatus 120 not experiencing a power outage indicates that the trolley has not been pushed out of the operating room, and therefore the first control apparatus 120 or the medical device 140 is determined to have not been moved out of the operating room.

Alternatively, the control system 100 may include an imaging device which displays that the first control apparatus 120 or the medical device 140 has not been moved out of the operating room since the last communicative connection between the medical device 140 and the control device 110 was established. For example, if the first control apparatus 120 or the medical device 140 is always within a shooting range of the imaging device inside the operating room, the first control apparatus 120 or the medical device 140 can be determined to never leave the operating room.

Alternatively, position information of the first control apparatus 120 and/or the medical device 140 can be compared with pre-stored map data of the operating room to determine that the first control apparatus 120 or the medical device 140 has not been moved out of the operating room. Wherein, indoor positioning technology can be used to obtain the position information of the first control apparatus 120 and/or the position information of the medical device 140.

In an example, if a communicative connection has been established between the medical device 140 and the control device 110, regardless of whether the scanning result indicates that the first control apparatus 120 is located inside or outside the first area, said communicative connection between the medical device 140 and the control device 110 is maintained until said communicative connection is disconnected. The control system 100 does not initiate to disconnect said communicative connection between the medical device 140 and the control device 110 just for a reason that the scanning result indicates that the first control apparatus 120 is located outside the first area. The reason for disconnecting the communicative connection may include a power failure of the medical device 140 or of the first control apparatus 120, or that the medical device 140 leaves a maximum coverage area of Wi-Fi signal.

In another example, if a communicative connection has been established between the medical device 140 and the control device 110, said communicative connection between the medical device 140 and the control device 110 can be maintained, when the scanning result indicates that the first control apparatus 120 is located outside the first area but inside the second area; and said communicative connection between the medical device 140 and the control device 110 can be disconnected, when the scanning result indicates that the first control apparatus 120 is located outside the second area. Wherein, the scanning result indicating that the first control apparatus 120 is located inside the second area, includes: the scanning result indicates that the distance between the first scanning module and the second scanning module is less than or equal to a second distance; or the scanning result indicates that the strength of the scanning signal is greater than or equal to a second signal strength.

According to the above strategy of communicative connection, it is possible to automatically establish a wireless connection with the control device 110 when the medical device 140 is moved into the operating room without requiring manual operation of user. The first area for establishing a communicative connection is limited to the operating room, which can prevent the medical device 140 from mistakenly connecting with other control devices in other operating rooms. The communicative connection is maintained, when the scanning result indicates that the medical device 140 is not inside the first area, which can avoid the problem of disconnection due to obstruction, movement to the corner of the operating room, or slight departure from the first area, and can improve the stability of communicative connection. If the communicative connection is accidentally disconnected, the communicative connection can be reestablished when the medical device 140 is determined to not leave the operating room, which solves the problem of accidental disconnection. In summary, the above strategy of communicative connection can achieve seamless connection and disconnection between the medical device 140 and the control device 110, as well as avoid the occurrence of accidental disconnection.

In another implementation, when the scanning result indicates that the first control apparatus 120 is located inside the first area, the medical device 140 establishes a communicative connection with the control device 110; when the scanning result indicates that the first control apparatus 120 is located outside the first area, and no communicative connection has been established between the medical device 140 and the control device 110, a wireless connection cannot be established between the medical device 140 and the control device 110. When there is no communicative connection between the medical device 140 and the control device 110, only when the scanning result indicates that the first control apparatus 120 is located inside the first area, a communicative connection will be established between the medical device 140 and the control device 110. The communicative connection would not be reestablished between the control device 110 and a medical device 140, which medical device is outside the first area.

If a communicative connection has been established between the medical device 140 and the control device 110, regardless of whether the scanning result indicates that the first control apparatus 120 is located inside or outside the first area, said communicative connection is maintained between the medical device 140 and the control device 110 until said communicative connection is disconnected. In the case that a communicative connection has been established between the medical device 140 and the control device 110, said communicative connection will not be disconnected for a reason that the scanning result indicates that the first control apparatus 120 is located outside the first area, so as to avoid affecting the stability of the communicative connection due to interference on the scanning signal or obstruction of the first control apparatus 120.

Based on the above analysis and determination on the scanning result, a communicative connection between the medical device 140 and the control device 110 can be established, disconnected, or maintained. The executing subject for analysing and determining the scanning result can be the second control apparatus 130 or the first control apparatus 120. Alternatively, when a communicative connection has been established between the second control apparatus 130 or the first control apparatus 120 and the control device 110, the second control apparatus 130 or the first control apparatus 120 can also transmit the scanning result to the control device 110. Then the control device 110 determines whether the scanning result between the second control apparatus 130 and the first control apparatus 120 satisfies the first condition or the second condition, or not, and then decides whether to establish, disconnect, or maintain a communicative connection between the medical device 140 and the control device 110.

According to the above strategy of communicative connection, it is possible to automatically establish a wireless connection with the control device 110 when the medical device 140 is moved into the operating room without requiring manual operation of user. The first area for establishing a communicative connection is limited to the operating room, which can prevent the medical device 140 from mistakenly connecting with other control devices in other operating rooms. The communicative connection is maintained, when the scanning result indicates that the medical device 140 is not inside the first area, which can avoid the problem of disconnection due to obstruction, movement to the corner of the operating room, or slight departure from the first area, and can improve the stability of communicative connection.

In summary, the above strategy of communicative connection can achieve seamless connection and disconnection between the medical device 140 and the control device 110, as well as avoid the occurrence of accidental disconnection. For example, when establishing a communicative connection between the medical device and the control device based on the scanning result, the first control apparatus 120 can be configured to implement relay communication between the medical device 140 and the control device 110, so as to establish a communicative connection between the medical device 140 and the control device 110. Relay communication refers to that the first control apparatus acts as a relay device to transmit to the medical device 140, in an unvarnished transmission mode, data which is downstream transmitted by the control device 110 to the medical device 140, and to transmit to the control device 140, in an unvarnished transmission mode, data which is upstream transmitted by the medical device 140 to the control device 110.

For example, if the medical device 140 is not equipped with a wireless communication module, that is, the medical device 140 does not have the ability to connect the wireless local area network on its own, the medical device 140 can be connected with the wired communication module of the first control apparatus 120, and at the same time, the wireless communication module of the first control apparatus 120 establishes a wireless communicative connection with the control device 110, thereby indirectly establishing a wireless communicative connection between the medical device 140 and the control device 110.

The first control apparatus 120 can establish a wireless connection with the control device 110 based on wireless connection information, wherein the wireless connection information is transmitted from the second control apparatus 130 to the first control apparatus 120, or the wireless connection information is pre-stored by the first control apparatus 120. Specifically, in the case that the first control apparatus 120 has not been connected with the wireless LAN before, the second control apparatus 130 can transmit wireless connection information for connecting the wireless LAN, such as Service Set Identifier (SSID) and password of the wireless LAN, to the first control apparatus 120. In the case that the first control apparatus 120 has been connected with the wireless LAN before, wireless connection information is usually stored in the first control apparatus 120. Therefore, the first control apparatus 120 can directly access the wireless LAN based on the pre-stored wireless connection information.

For the case that the control device 110 and the medical device 140 perform relay communication through the first control apparatus 120, the control device 110 can initiate a request for establishing a communicative connection, so as to establish the communicative connection between the control device 110 and the medical device 140 through relay communication via the first control apparatus 120. Alternatively, the medical device 140 can initiate a request for establishing a communicative connection, so as to establish the communicative connection between the control device 110 and the medical device 140 through relay communication via the first control apparatus 120. Alternatively, the first control apparatus 120 can respectively transmit a request for establishing a communicative connection to the control device 110 and the medical device 140, so as to establish the communicative connection between the control device 110 and the first control apparatus 120, as well as between the medical device 140 and the first control apparatus 120. Alternatively, the control device 110, the medical device 140, and the first control apparatus 120 can jointly maintain a communicative connection among the three parties.

For the case that the first control apparatus 120 serves as a communication relay device between the control device 110 and the medical device 140, when it is necessary to disconnect the communicative connection between the medical device 140 and the control device 110, as long as either or both of the communication link/links between the first control apparatus 120 and the medical device 140, as well as between the first control apparatus 120 and the control device 110, is/are disconnected, the communicative connection between the medical device 140 and the control device 110 can be disconnected. When the communicative connection is established between the first control apparatus 120 and the medical device 140 through wired means, the first control apparatus 120 can disconnect the communicative connection with the medical device 140 by disabling a corresponding communication interface. When the communicative connection is established wirelessly between the first control apparatus 120 and the medical device 140, the first control apparatus 120 can disconnect the communicative connection with the medical device 140 by disabling transmission and reception of wireless signal with the medical device 140.

In an example, the wireless connection between the first control apparatus 120 and the control device 110 can be disconnected via the first control apparatus 120. Wherein, the second control apparatus 130 instructs the first control apparatus 120 to disconnect the wireless connection, or the first control apparatus 120 initiate to disconnect the wireless connection, or the control device 110 instructs the first control apparatus 120 to disconnect the wireless connection. Alternatively, the communicative connection between the control device 110 and the first control apparatus 120 can be disconnected by the control device 110 or the medical device 140.

If the medical device 140 is equipped with a wireless communication module, that is, it has the ability to access the wireless local area network on its own without requiring to communicate with the control device 110 through using the first control apparatus 120 as a relay device. Then when establishing the communicative connection between the medical device 140 and the control device 110, the first control apparatus 120 can transmit wireless connection information to the medical device 140, so that the medical device 140 can establish a wireless connection with the control device 110 based on the wireless connection information.

Specifically, when the medical device 140 is connected with the wireless LAN in the operating room at a first time, the first control apparatus 120 needs to transmit wireless connection information which is used to connect the wireless LAN, such as Service Set Identifier (SSID) and password, to the medical device 140, so as to enable the medical device 140 to be successfully connected with the wireless LAN. After obtaining SSID and other information for connecting the wireless LAN, the medical device 140 can initiate a connection request to the control device 110 based on the SSID and other information, so as to connect with the wireless LAN.

Wherein, the wireless connection information is transmitted from the second control apparatus 130 to the first control apparatus 120, or the wireless connection information is prestored by the first control apparatus 120. Usually, in the case that the first control apparatus 120 has not been connected with the wireless LAN, the second control apparatus 130 can transmit wireless connection information to the first control apparatus 120. In the case that the first control apparatus 120 has been connected with the wireless LAN before, the wireless connection information is usually stored in the first control apparatus 120, so the pre-stored wireless connection information can be directly transmitted to the medical device 140.

For example, the second control apparatus 130 can transmit the wireless connection information to the first scanning module through the second scanning module. For example, when the first scanning module and the second scanning module are UWB modules, the second control apparatus 130 can transmit wireless connection information to the first control apparatus 120 through a UWB signal.

When the control device 110 directly communicates with the medical device 140, the control device 110 can initiate a request for establishing a communicative connection to establish the communicative connection between the control device 110 and the medical device 140. Alternatively, the request for establishing a communicative connection can be initiated by the medical device 140 to establish the communicative connection between control device 110 and medical device 140. Alternatively, the control device 110 and the medical device 140 can jointly maintain the communicative connection between both parties.

In the case that the control device 110 directly communicates with the medical device 140, the first control apparatus 120 can instruct the medical device to disconnect the wireless connection with the control device. Alternatively, the control device 110 can initiate to disconnect the communicative connection between the control device 110 and the medical device 140. Alternatively, the medical device 140 can initiate to disconnect the communicative connection between the control device 110 and the medical device 140.

In summary, the control system of embodiments of this disclosure establishes, disconnects, or maintains a communicative connection between the medical device and the control device based on a scanning result between the first scanning module and the second scanning module. When the scanning result satisfies the first condition, the communicative connection between the medical device and the control device is established. When the scanning result does not satisfy the first condition, the communicative connection between the medical device and the control device is not disconnected, thereby avoiding the problem of unstable communicative connection caused by signal obstruction and other reasons.

In the embodiment of this disclosure, firstly, based on the second scanning module which is configured in the second control apparatus, and the first scanning module which is configured in the first control apparatus, scanning is performed to determine that whether the scanning result satisfies a condition. The condition here can be the first condition, the second condition, etc. in the above embodiment; or can be inside the first area, outside the first area, inside the second area, or outside the second area in the above embodiments; or inside or outside the first area as described in the above embodiments. Secondly, based on the scanning result, the communicative connection between the control device and the medical device can be established/maintained/disconnected. Here, it should be noted that the executing entity responsible for determining whether the scanning result satisfies the condition(s), and the initiator to establish/maintain/disconnect the communicative connection between the control device and the medical device, can be the same or different.

Specifically, for the case that the executing entity which determines whether the scanning result satisfies the condition, and the initiator which establishes/maintains/disconnects the communicative connection between the control device and the medical device, are the same, there are following specific situations.
1. The first control apparatus determines whether the scanning result satisfies the condition, and acts as the initiator to establish/maintain/disconnect the communicative connection between the control device and the medical device.
2. The control device determines whether the scanning result satisfies the condition, and acts as the initiator to establish/maintain/disconnect the communicative connection between the control device and the medical device.

In addition, for the case that the second control apparatus determines whether the scanning result satisfies the condition and acts as the initiator to establish/maintain/disconnect the communicative connection between the control device and the medical device, it can be achieved in the scenario of wireless LAN communication between the control device and the medical device. At this point, the second control apparatus can transmit wireless connection information for connecting with the wireless LAN to the corresponding first control apparatus, so that the first control apparatus can forward the wireless connection information to the relevant medical device, thereby enabling the medical device to connect with the wireless LAN. Therefore, it can also be equivalent to that the first control apparatus initiates establishment/maintenance/disconnection of the communicative connection between the control device and the medical device.

For the case that the executing entity which determines whether the scanning result satisfies the condition, and the initiator which establishes/maintains/disconnects the communicative connection between the control device and the medical device, are different, there are following specific situations.
1. The first control apparatus determines whether the scanning result satisfies the condition, and the control device or the medical device acts as the initiator to establish/maintain/disconnect the communicative connection between the control device and the medical device. At this time, the first control apparatus can transmit a determination result to the control device, so as to enable the control device to act as the initiator to establish/maintain/disconnect the communicative connection between the control device and the medical device. Alternatively, the first control apparatus can instruct the medical device to initiate/maintain/disconnect the communicative connection between the control device and the medical device through an established communicative connection with the medical device.
2. The second control apparatus determines whether the scanning result satisfies the condition, and the control device or the medical device or the first control apparatus acts as the initiator to establish/maintain/disconnect the communicative connection between the control device and the medical device. At this time, the second control apparatus can transmit a determination result to the control device, so as to enable the control device to act as the initiator to establish/maintain/disconnect the communicative connection between the control device and the medical device. Alternatively, the second control apparatus can transmit the determination result to the first control apparatus, so as to enable the first control apparatus to act as the initiator to establish/maintain/disconnect the communicative connection between the control device and the medical device. For the case that the second control apparatus determines whether the scanning result satisfies the condition and the medical device acts as the initiator to establish/maintain/disconnect the communicative connection between the control device and the medical device, the second control apparatus needs to transmit the determination result to the first control apparatus, and then the first control apparatus instructs the medical device to initiate to establish/maintain/disconnect the communicative connection between the control device and the medical device.
3. The control device determines whether the scanning result satisfies the condition, and the medical device or the first control apparatus acts as the initiator to establish/maintain/disconnect the communicative connection between the control device and the medical device. At this time, the control device can transmit the determination result to the first control apparatus, so as to enable the first control apparatus to act as the initiator to establish/maintain/disconnect the communicative connection between the control device and the medical device. For the case that the control device determines whether the scanning result satisfies the condition and the medical device acts as the initiator to establish/maintain/disconnect the communicative connection between the control device and the medical device, the control device needs to transmit the determination result to the first control apparatus through the first communication module, and the first control apparatus instructs the medical device to initiate to establish/maintain/disconnect the communicative connection between the control device and the medical device.

Optionally, based on the aforementioned embodiments, in a possible implementation, the establishment process of relay communication can be initiated by the control device, which transmits to the first control apparatus a request for establishing a communicative connection. After receiving the request for establishing a communicative connection, the first control apparatus establishes communication links with both the control device and the medical device, thereby achieving the communicative connection through the first control apparatus as communication relay. Alternatively, the establishment process of relay communication can be initiated by the medical device, which transmits to the first control apparatus a request for establishing a communicative connection. After receiving the request for establishing a communicative connection, the first control apparatus establishes communication links with both the control device and the medical device, thereby achieving the communicative connection through the first control apparatus as a communication relay. Alternatively, the first control apparatus can simultaneously transmits requests for establishing a communicative connection to both the control device and the medical device, so as to establish a direct communicative connection between the control device and the first control apparatus, as well as a direct communicative connection between the medical device and the first control apparatus.

Disconnecting the communicative connection between the medical device and the control device through the first control apparatus as a communication relay, can be initiated by the control device, so as to disconnect or prohibit the communicative connection between the control device and the first control apparatus; alternatively, can be initiated by the control device, so as to disconnect or prohibit communication between the control device and the first control apparatus based on network layer protocols; alternatively, can be disconnecting or prohibiting message forwarding from the control device to the medical device at an application layer of the control device; alternatively, can be disconnecting or prohibiting message transmission to the first control apparatus at an application layer of the control device; alternatively, can be ignoring message(s) forwarded by the first control apparatus from the medical device at an application layer of the control device.

Alternatively, disconnecting the communicative connection between the medical device and the control device through the first control apparatus as a communication relay, can be initiated by the medical device, so as to disconnect or prohibit the communicative connection between the medical device and the first control apparatus; alternatively, can be initiated by the medical device, so as to disconnect or prohibit communication between the medical device and the first control apparatus based on network layer protocols; alternatively, can be disconnecting or prohibiting message forwarding from the medical device to the control device at an application layer of the medical device; alternatively, can be disconnecting or prohibiting message transmission to the first control apparatus at an application layer of the medical device; alternatively, can be ignoring message(s) forwarded by the first control apparatus from the control device at an application layer of the medical device.

Alternatively, disconnecting the communicative connection between the medical device and the control device through the first control apparatus as a communication relay, can be initiated by the first control apparatus, so as to disconnect or prohibit the communicative connection between the control device and the first control apparatus; alternatively, can be initiated by the first control apparatus, so as to disconnect or prohibit the communicative connection between the medical device and the first control apparatus; alternatively, can be initiated by the first control apparatus, so as to disconnect or prohibit communication between the first control apparatus and the medical device based on network layer protocols; alternatively, can be initiated by the first control apparatus, so as to disconnect or prohibit communication between the first control apparatus and the control device based on network layer protocols; alternatively, can be disconnecting or prohibiting message forwarding from the control device to the medical device at an application layer of the first control apparatus; alternatively, can be disconnecting or prohibiting message forwarding from the medical device to the control device at an application layer of the first control apparatus; alternatively, can be disconnecting or prohibiting message transmission to the medical device or the control device at an application layer of the first control apparatus; alternatively, can be ignoring message(s) transmitted from the control device or the medical device at an application layer of the first control apparatus.

That is to say, in the case that the first control apparatus serves as a relay communication device, as long as either or both of communication links between the first control apparatus and the medical device, and between the first control apparatus and the control device, is/are disconnected, the communicative connection between the control device and the medical device can be disconnected.

Next, a method for controlling connection of an operating room according to various embodiments of this disclosure is described with reference to FIGS. 4 to 6. The method for controlling connection in this embodiment can be implemented in the control system 100 for an operating room described above. Specifically, each step of the method for controlling connection of an operating room can be executed by the control device 110, the first control apparatus 120, or the second control apparatus 130 of the control system 100 for an operating room.

Firstly, referring to FIG. 4, a method 400 for controlling connection of an operating room in one embodiment of this disclosure includes following steps.

In step S410, obtain a scanning result between the first control apparatus and the second control apparatus.

In step S420, when the scanning result satisfies a first condition and no communicative connection has been established between the medical device and the control device, establish a communicative connection between the medical device and the control device.

In step S430, when the scanning result does not satisfy the first condition and no communicative connection has been established between the medical device and the control device, maintain no communicative connection between the medical device and the control device.

In step S440, when the scanning result satisfies the first condition or satisfies a second condition and a communicative connection has been established between the medical device and the control device, maintain said communicative connection between the medical device and the control device.

In step S450, when the scanning result does not satisfy the first condition and the second condition and a communicative connection has been established between the medical device and the control device, disconnect said communicative connection between the medical device and the control device.

When the scanning result indicates a distance, a maximum distance which satisfies the first condition, is less than a maximum distance which satisfies the second condition; or when the scanning result indicates a strength of a scanning signal, a minimum signal strength which satisfies the first condition is greater than a minimum signal strength which satisfies the second condition; or when the scanning result indicates a position of the first control apparatus, an area which corresponds to a possible position of the first control apparatus, which position satisfies the first condition, is less than an area which corresponds to a possible position of the first control apparatus, which position satisfies the second condition.

For example, the scanning result satisfying the first condition indicates that a distance between the first control apparatus and the second control apparatus is less than or equal to a first distance; the scanning result satisfying the second condition indicates that the distance between the first control apparatus and the second control apparatus is less than or equal to a second distance; wherein the first distance is less than the second distance; or
the scanning result satisfying the first condition indicates that a distance between the first control apparatus and the second control apparatus is less than or equal to a first distance; the scanning result satisfying the second condition indicates that the distance between the first control apparatus and the second control apparatus is greater than a third distance but less than or equal to a second distance; wherein the first distance is less than the second distance, the third distance is not greater than the first distance; or
the scanning result satisfying the first condition indicates that a strength of the scanning signal is greater than or equal to a first signal strength; the scanning result satisfying the second condition indicates that the strength of the scanning signal is greater than or equal to a second signal strength; wherein the first signal strength is greater than the second signal strength; or
the scanning result satisfying the first condition indicates that a strength of the scanning signal is greater than or equal to a first signal strength; the scanning result satisfying the second condition indicates that the strength of the scanning signal is less than a third signal strength but greater than or equal to a second signal strength; wherein the third signal strength is not less than the first signal strength, the first signal strength is greater than the second signal strength.

For example, the scanning result satisfying the first condition indicates that the first control apparatus is located inside a first area which is inside the operating room and capable of being scanned by the second scanning module; the scanning result satisfying the second condition indicates that the first control apparatus is located inside a second area which is capable of being scanned by the second scanning module; wherein the second area covers the operating room entirely, or the second area covers the first area and is greater than the first area.

The medical device and the control device can perform relay communication through the first control apparatus, wherein establishing a communicative connection between the medical device and the control device includes: establishing, by the first control apparatus, a wireless connection between the control device and the first control apparatus, based on wireless connection information which is transmitted from the second control apparatus to the first control apparatus or pre-stored by the first control apparatus.

In the case that the first control apparatus provides relay communication, disconnecting said communicative connection between the medical device and the control device includes: disconnecting, by the first control apparatus, a wireless connection between the first control apparatus and the control device, wherein the second control apparatus instructs the first control apparatus to disconnect the wireless connection, or the first control apparatus initiate to disconnects the wireless connection.

When the medical device is equipped with a wireless communication module, it can also directly establish a communicative connection with the control device. At this time, establishing a communicative connection between the medical device and the control device includes: transmitting, by the first control apparatus, wireless connection information to the medical device, so as to enable the medical device to establish the wireless connection with the control device based on the wireless connection information; wherein, the wireless connection information of the medical device is transmitted from the second control apparatus to the first control apparatus, or the wireless connection information is pre-stored by the first control apparatus.

In the case that the medical device is in a direct communicative connection with the control device, disconnecting said communicative connection between the medical device and the control device, includes instructing, by the first control apparatus, the medical device to disconnect said wireless connection between the medical device and the control device.

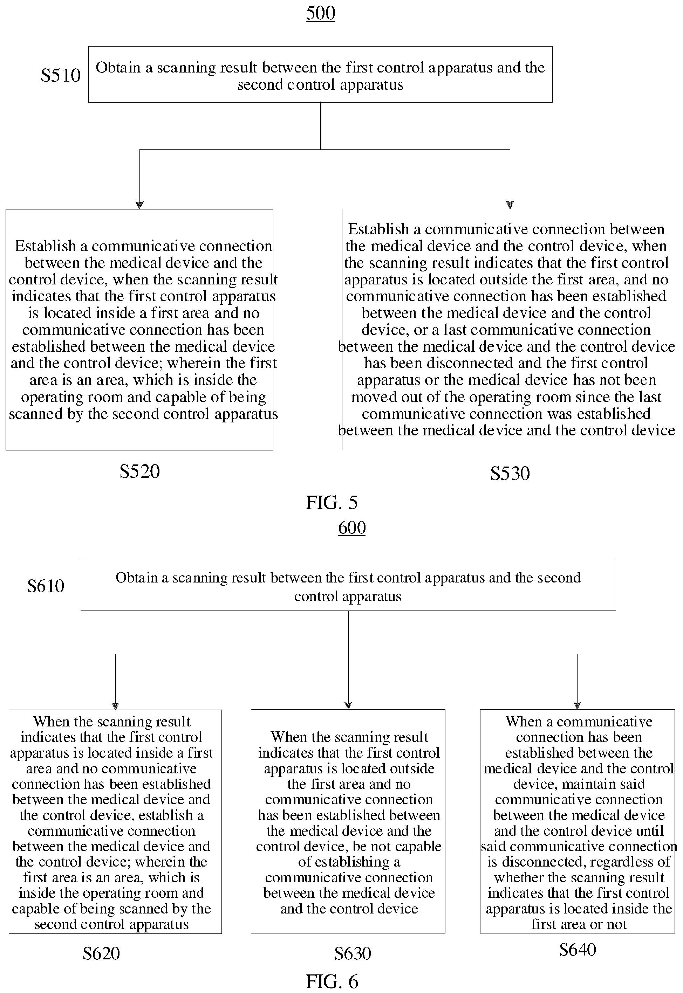

Referring to FIG. 5, a method 500 for controlling connection of an operating room in one embodiment of this disclosure includes following steps.

In step S510, obtain a scanning result between the first control apparatus and the second control apparatus.

In step S520, establish a communicative connection between the medical device and the control device, when the scanning result indicates that the first control apparatus is located inside a first area and no communicative connection has been established between the medical device and the control device; wherein the first area is an area, which is inside the operating room and capable of being scanned by the second control apparatus.

In step S530, establish a communicative connection between the medical device and the control device, when the scanning result indicates that the first control apparatus is located outside the first area, and no communicative connection has been established between the medical device and the control device, or a last communicative connection between the medical device and the control device has been disconnected and the first control apparatus or the medical device has not been moved out of the operating room since the last communicative connection was established between the medical device and the control device.

For example, the scanning result indicates that the first control apparatus is located inside a first area, includes: the scanning result indicates that a distance between the first control apparatus and the second control apparatus is less than or equal to a first distance; or the scanning result indicates that the first control apparatus is located inside the first area which is inside the operating room and capable of being scanned by the second control apparatus; or the scanning result indicates that a strength of a scanning signal is greater than or equal to a first signal strength;
the scanning result indicates that the first control apparatus is located outside the first area, includes: the scanning result indicates that the distance between the first control apparatus and the second control apparatus is greater than the first distance; or the scanning result indicates that the first control apparatus is located outside the first area which is inside the operating room and capable of being scanned by the second control apparatus; or the scanning result indicates that the strength of the scanning signal is less than the first signal strength.

For example, the first control apparatus or the medical device having not been moved out of the operating room since the last communicative connection was established between the medical device and the control device, includes: the first control apparatus has not experienced a power outage, since the last communicative connection was established between the medical device and the control device; the control system further includes an imaging device, which displays that the first control apparatus or the medical device has not been moved out of the operating room since the last communicative connection was established between the medical device and the control device; or position information of the first control apparatus and/or of the medical device is compared with pre-stored map data of the operating room to determine that the first control apparatus or the medical device has not been moved out of the operating room since the last communicative connection was established between the medical device and the control device.

For example, when a communicative connection has been established between the medical device and the control device; maintain said communicative connection between the medical device and the control device until said communicative connection is disconnected, regardless of whether the scanning result indicates that the first control apparatus is located inside the first area or not.

For example, when a communicative connection has been established between the medical device and the control device; maintain said communicative connection between the medical device and the control device, when the scanning result indicates that the first control apparatus is located inside the first area; maintain said communicative connection between the medical device and the control device, when the scanning result indicates that the first control apparatus is located outside the first area but inside the second area; and disconnect said communicative connection between the medical device and the control device, when the scanning result indicates that the first control apparatus is located outside the second area.

For example, if the scanning result indicates that the first control apparatus is located outside the first area but inside the second area; establish a communicative connection between the first control apparatus and the control device, when no communicative connection has been established between the medical device and the control device, or a last communicative connection between the medical device and the control device has been disconnected and the first control apparatus or the medical device has not been moved out of the operating room since the last communicative connection was established between the medical device and the control device.

Referring to FIG. 6, a method 600 for controlling connection of an operating room in one embodiment of this disclosure includes following steps.

In step S610, obtain a scanning result between the first control apparatus and the second control apparatus.

In step S620, when the scanning result indicates that the first control apparatus is located inside a first area and no communicative connection has been established between the medical device and the control device, establish a communicative connection between the medical device and the control device; wherein the first area is an area, which is inside the operating room and capable of being scanned by the second control apparatus.

In step S630, when the scanning result indicates that the first control apparatus is located outside the first area and no communicative connection has been established between the medical device and the control device, be not capable of establishing a communicative connection between the medical device and the control device.

In step S640, when a communicative connection has been established between the medical device and the control device, maintain said communicative connection between the medical device and the control device until said communicative connection is disconnected, regardless of whether the scanning result indicates that the first control apparatus is located inside the first area or not.

For example, the scanning result indicating that the first control apparatus is located inside a first area, includes: the scanning result indicates that a distance between the first control apparatus and the second control apparatus is less than or equal to a first distance; or the scanning result indicates that the first control apparatus is located inside the first area which is inside the operating room and capable of being scanned by the second control apparatus; or the scanning result indicates that a strength of a scanning signal between the first control apparatus and the second control apparatus is greater than or equal to a first signal strength;
the scanning result indicating that the first control apparatus is located outside the first area, includes: the scanning result indicates that the distance between the first control apparatus and the second control apparatus is greater than the first distance; or the scanning result indicates that the first control apparatus is located outside the first area which is inside the operating room and capable of being scanned by the second control apparatus; or the scanning result indicates that the strength of the scanning signal between the first control apparatus and the second control apparatus is less than the first signal strength.

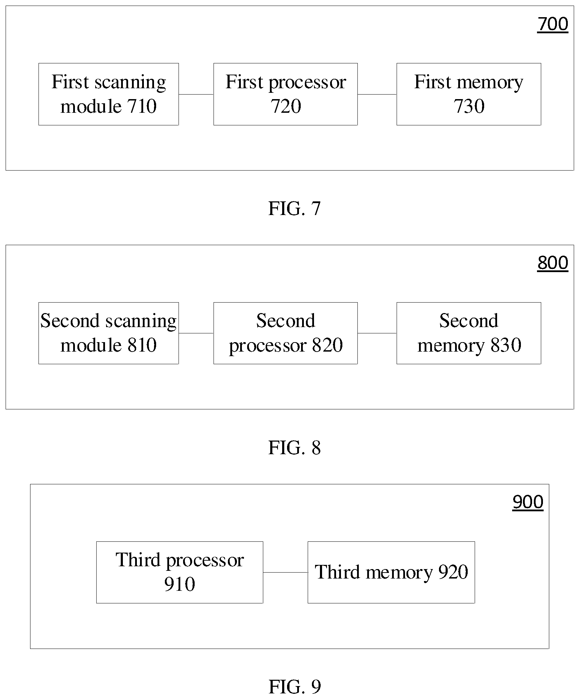

As shown in FIG. 7, an embodiment of this disclosure also provides a control apparatus 700, which is used for a control system for an operating room, and can be specifically implemented as the first control apparatus in the control system 100 described above. The control apparatus 700 has established a communicative connection with the medical device, and a position of the control apparatus 700 changes synchronously with a position of the medical device. The control apparatus 700 includes a first scanning module 710, a first processor 720, and a first memory 730, which stores a computer program which is executed by the first processor 720, wherein the above method for controlling connection is capable of being implemented when the computer program is executed by the first processor 720.

For example, the first scanning module 710 includes a UWB module, a Wi-Fi module, an infrared module, and a Bluetooth module. The first scanning module 710 is configured to transmit and/or receive a scanning signal, so as to obtain a scanning result, which can be used to indicate a distance, signal strength, or a position of a medical device.

The first processor 720 can be a general-purpose processor, a digital signal processor (DSP), or other programmable logic devices, discrete gate or transistor logic devices, discrete hardware components, etc. The first processor 720 is capable of implementing or executing the disclosed methods, steps, and logic diagrams in the embodiments of this disclosure. A general-purpose processor can be a microprocessor or any conventional processor, etc. The steps of the method disclosed in the embodiments of this disclosure can be directly reflected as being executed by a hardware decoding processor, or as being executed by a combination of hardware and software modules in the decoding processor. The software module can be located in a storage medium, which is located inside the first memory 730. The first processor 720 reads information inside the first memory 730 and completes steps of the aforementioned method in combination with its hardware.

As shown in FIG. 8, an embodiment of this disclosure also provides a control apparatus 800, which is used for a control system for an operating room and can be specifically implemented as the second control apparatus in the control system 100 described above. The control apparatus 800 is arranged at a designated position of the operating room. The control apparatus 800 includes a second scanning module 810, a second processor 820, and a second memory 830. The second memory 830 stores a computer program which is executed by the second processor 820, wherein the above method for controlling connection is capable of being implemented when the computer program is executed by the second processor 820.

For example, the second scanning module 810 includes a UWB module, a Wi-Fi module, an infrared module, and a Bluetooth module. The second scanning module 810 is configured to transmit and/or receive a scanning signal, so as to obtain a scanning result, which can be used to indicate a distance, signal strength, or a position of a medical device.

The second processor 820 can be a general-purpose processor, a digital signal processor (DSP), or other programmable logic devices, discrete gate or transistor logic devices, discrete hardware components, etc. The second processor 820 is capable of implementing or executing the disclosed methods, steps, and logic diagrams in the embodiments of this disclosure. A general-purpose processor can be a microprocessor or any conventional processor, etc. The steps of the method disclosed in the embodiments of this disclosure can be directly reflected as being executed by a hardware decoding processor, or as being executed by a combination of hardware and software modules in the decoding processor. The software module can be located in a storage medium, which is located inside the second memory 830. The second processor 820 reads information in the second memory 830 and completes steps of the aforementioned method in combination with its hardware.

As shown in FIG. 9, an embodiment of this disclosure also provides a control device 900 for an operating room, which includes a third processor 910 and a third memory 920. The third memory 920 stores a computer program which is executed by the third processor 910, wherein the above method for controlling connection or the following control method for a control device for an operating room is capable of being implemented when the computer program is executed by the third processor 910.

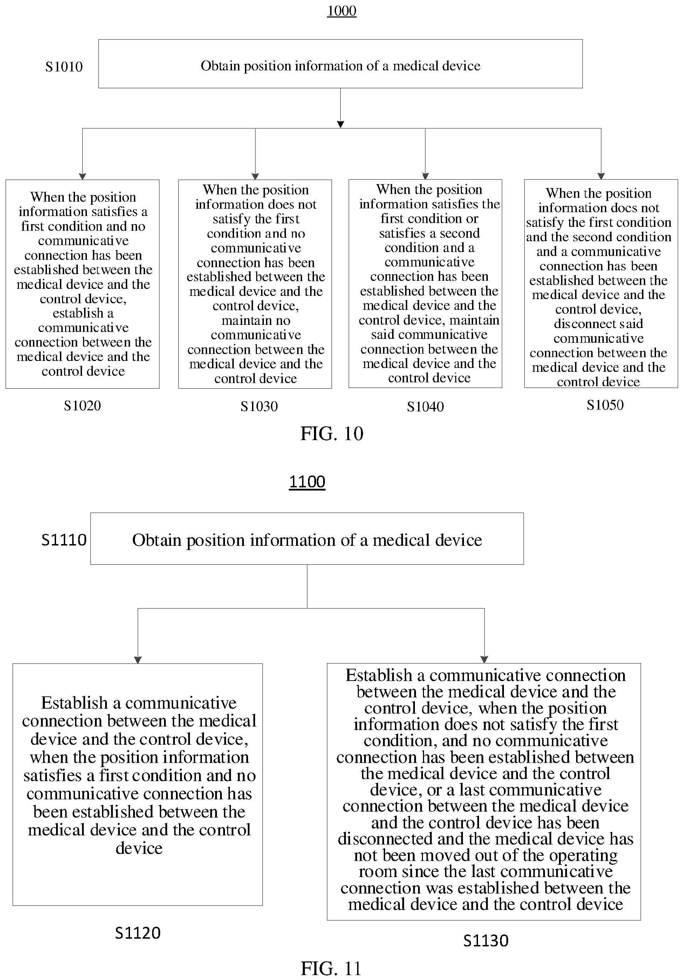

As shown in FIG. 10, a control method 1000 for a control device for an operating room according to an embodiment of this disclosure includes following steps.

In step S1010, obtain position information of a medical device;
In step S 1020, when the position information satisfies a first condition and no communicative connection has been established between the medical device and the control device, establish a communicative connection between the medical device and the control device.

In step S 1030, when the position information does not satisfy the first condition and no communicative connection has been established between the medical device and the control device, maintain no communicative connection between the medical device and the control device.

In step S1040, when the position information satisfies the first condition or satisfies a second condition and a communicative connection has been established between the medical device and the control device, maintain said communicative connection between the medical device and the control device.

In step S 1050, when the position information does not satisfy the first condition and the second condition and a communicative connection has been established between the medical device and the control device, disconnect said communicative connection between the medical device and the control device.

Wherein an area which corresponds to a possible position of the medical device, which position satisfies the first condition, is less than an area which corresponds to a possible position of the medical device, which position satisfies the second condition. The position information of the medical device can be obtained through positioning technology or through an imaging device. For example, the position information of the medical device can be obtained through indoor positioning technology, and the position information of the medical device can be compared with map data of the operating room, so as to determine that whether the position information satisfies the first condition or the second condition. Alternatively, the medical device can be identified in the image data acquired by the imaging device, and then the position information of the medical device can be obtained based on a recognition result of the medical device.

In one embodiment, the position information satisfying the first condition indicates that the medical device is located inside a first area, which is inside the operating room; the position information satisfying the second condition indicates that the medical device is located inside a second area, which covers the operating room entirely, or covers the first area and is greater than the first area.

The control method 1000 for a control device for an operating room based on the embodiment of this disclosure can enable the medical device to automatically establish a wireless connection with the control device, when the position information of the medical device satisfies the first condition, without requiring manual operation of user; to maintain a communicative connection with the control device when the position information does not satisfy the first condition, thus avoiding the problem of disconnection due to obstruction, movement to the corner of the operating room, or slight departure from a preset area, and improving the stability of communicative connection. Furthermore, setting the second condition for disconnection can achieve automatic disconnection of the medical device after leaving the operating room. In summary, the control method 1000 for a control device for an operating room based on the embodiment of this disclosure can achieve seamless connection and disconnection between the medical device and the control device, as well as avoid the occurrence of accidental disconnection.

As shown in FIG. 11, a control method 1100 for a control device for an operating room according to an embodiment of this disclosure includes following steps.

In step S 1110, obtain position information of a medical device;
In step S 1120, establish a communicative connection between the medical device and the control device, when the position information satisfies a first condition and no communicative connection has been established between the medical device and the control device.

In step S1130, establish a communicative connection between the medical device and the control device, when the position information does not satisfy the first condition, and no communicative connection has been established between the medical device and the control device, or a last communicative connection between the medical device and the control device has been disconnected and the medical device has not been moved out of the operating room since the last communicative connection was established between the medical device and the control device.

In one embodiment, the medical device having not been moved out of the operating room since the last communicative connection was established between the medical device and the control device, includes: the medical device has not experienced a power outage, since the last communicative connection was established between the medical device and the control device; an imaging device displays that the medical device has not been moved out of the operating room, since the last communicative connection was established between the medical device and the control device; position information of the medical device is compared with pre-stored map data of the operating room to determine that the medical device has not been moved out of the operating room since the last communicative connection was established between the medical device and the control device.

In one embodiment, when a communicative connection has been established between the medical device and the control device, the method further includes maintaining said communicative connection between the medical device and the control device until said communicative connection is disconnected, regardless of whether the position information indicates that the medical device is located inside the first area or not; wherein the first area is an area inside the operating room.

For example, in the case that a communicative connection has been established between the medical device and the control device; when the position information satisfies the first condition or satisfies the second condition, maintain the communicative connection between the medical device and the control device; when the position information does not satisfy the second condition, disconnect the communicative connection between the medical device and the control device; wherein, an area which corresponds to a possible position of the medical device, which position satisfies the first condition, is less than an area which corresponds to a possible position of the medical device, which position satisfies the second condition.

Optionally, satisfying the first condition indicates that the medical device is located inside the first area, not satisfying the first condition indicates that the medical device is located outside the first area, satisfying the second condition indicates that the medical device is located inside the second area, and not satisfying the second condition indicates that the medical device is located outside the second area. The second area covers the operating room entirely, or the second area covers the first area and is greater than the first area.

The control method 1100 for a control device for an operating room based on the embodiment of this disclosure can enable the medical device to automatically establish a wireless connection with the control device, when the position information of the medical device satisfies the first condition, without requiring manual operation of user; to maintain a communicative connection with the control device when the position information does not satisfy the first condition, thus avoiding the problem of disconnection due to obstruction, movement to the corner of the operating room, or slight departure from a preset area, and improving the stability of communicative connection. Furthermore, if the communicative connection is accidentally disconnected, it can be reestablished when determining that the medical device has not left the operating room, which solves the problem of accidental disconnection. In summary, the control method 1100 for a control device for an operating room based on the embodiment of this disclosure can achieve seamless connection and disconnection between the medical device and the control device, as well as avoid the occurrence of accidental disconnection.

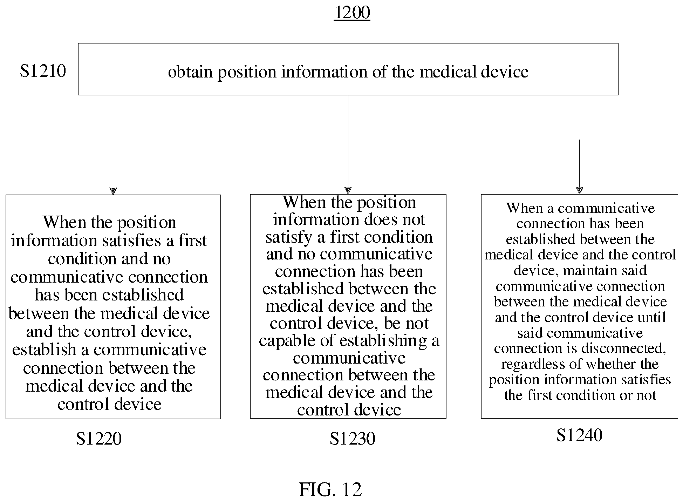

As shown in FIG. 12, a control method 1200 for a control device for an operating room according to an embodiment of this disclosure includes following steps.

In step S1210, obtain position information of the medical device.

In step S1220, when the position information satisfies a first condition and no communicative connection has been established between the medical device and the control device, establish a communicative connection between the medical device and the control device.

In step S1230, when the position information does not satisfy a first condition and no communicative connection has been established between the medical device and the control device, be not capable of establishing a communicative connection between the medical device and the control device.

In step S1240, when a communicative connection has been established between the medical device and the control device, maintain said communicative connection between the medical device and the control device until said communicative connection is disconnected, regardless of whether the position information satisfies the first condition or not.

Optionally, satisfying the first condition indicates that the medical device is located inside the first area, not satisfying the first condition indicates that the medical device is located outside the first area, wherein the first area is an area inside the operating room.

The control method 1200 for a control device for an operating room based on the embodiment of this disclosure can enable the medical device to automatically establish a wireless connection with the control device, when the position information of the medical device satisfies the first condition, without requiring manual operation of user; to maintain a communicative connection with the control device when the position information does not satisfy the first condition, thus avoiding the problem of disconnection due to obstruction, movement to the corner of the operating room, or slight departure from a preset area, and improving the stability of communicative connection. In summary, the control method 1200 for a control device for an operating room based on the embodiment of this disclosure can achieve seamless connection and disconnection between the medical device and the control device, as well as avoid the occurrence of accidental disconnection.

More specific details of the method for controlling connection of an operating room, the control apparatus, the control method for a control device for an operating room, and the control device for an operating room in embodiments of this disclosure, can refer to the control system 100 for an operating room mentioned above, and are not repeated here.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in an electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated to another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, in addition to mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and ordinary technical personnel in this field can design alternative embodiments without departing from the scope of the attached claims. In the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. A control system for an operating room, **characterized in that**, comprising: a control device for the operating room, a first control apparatus, and a second control apparatus;
the first control apparatus is in communicative connection with a medical device, wherein the first control apparatus comprises a first scanning module, and a position of the first control apparatus changes synchronously with a position of the medical device;
the second control apparatus is arranged at a designated position of the operating room inside which room the control device is located, wherein the second control apparatus comprises a second scanning module; the first scanning module is configured to receive a scanning signal which is transmitted by the second scanning module and/or the second scanning module is configured to receive a scanning signal which is transmitted by the first scanning module, so as to obtain a scanning result;
wherein:
when the scanning result satisfies a first condition and no communicative connection has been established between the medical device and the control device, a communicative connection is established between the medical device and the control device;
when the scanning result does not satisfy the first condition and no communicative connection has been established between the medical device and the control device, no communicative connection between the medical device and the control device is maintained;
when the scanning result satisfies the first condition or satisfies a second condition and a communicative connection has been established between the medical device and the control device, said communicative connection is maintained between the medical device and the control device;
when the scanning result does not satisfy the first condition and the second condition and a communicative connection has been established between the medical device and the control device, said communicative connection between the medical device and the control device is disconnected;
wherein, when the scanning result indicates a distance, a maximum distance which satisfies the first condition, is less than a maximum distance which satisfies the second condition; or
when the scanning result indicates a strength of the scanning signal, a minimum signal strength which satisfies the first condition is greater than a minimum signal strength which satisfies the second condition; or
when the scanning result indicates a position of the first scanning module, an area which corresponds to a possible position of the first scanning module, which position satisfies the first condition, is less than an area which corresponds to a possible position of the first scanning module, which position satisfies the second condition.

2. The control system according to claim 1, **characterized in that**, the scanning result satisfying the first condition indicates that a distance between the first scanning module and the second scanning module is less than or equal to a first distance; the scanning result satisfying the second condition indicates that the distance between the first scanning module and the second scanning module is less than or equal to a second distance; wherein the first distance is less than the second distance; or
the scanning result satisfying the first condition indicates that a distance between the first scanning module and the second scanning module is less than or equal to a first distance; the scanning result satisfying the second condition indicates that the distance between the first scanning module and the second scanning module is greater than a third distance but less than or equal to a second distance; wherein the first distance is less than the second distance, the third distance is not greater than the first distance; or
the scanning result satisfying the first condition indicates that a strength of the scanning signal is greater than or equal to a first signal strength; the scanning result satisfying the second condition indicates that the strength of the scanning signal is greater than or equal to a second signal strength; wherein the first signal strength is greater than the second signal strength; or
the scanning result satisfying the first condition indicates that a strength of the scanning signal is greater than or equal to a first signal strength; the scanning result satisfying the second condition indicates that the strength of the scanning signal is less than a third signal strength but greater than or equal to a second signal strength; wherein the third signal strength is not less than the first signal strength, the first signal strength is greater than the second signal strength.

3. The control system according to claim 1, **characterized in that**, the scanning result satisfying the first condition indicates that the first control apparatus is located inside a first area which is inside the operating room and capable of being scanned by the second scanning module;
the scanning result satisfying the second condition indicates that the first control apparatus is located inside a second area which is capable of being scanned by the second scanning module;
wherein the second area covers the operating room entirely, or the second area covers the first area and is greater than the first area.

4. The control system according to claim 1, **characterized in that**,
a communicative connection being established between the medical device and the control device, comprises: establishing, by the first control apparatus, a wireless connection between the control device and the first control apparatus, based on wireless connection information; and performing, by the first control apparatus, relay communication between the medical device and the control device; wherein the wireless connection information is transmitted from the second control apparatus to the first control apparatus, or is pre-stored by the first control apparatus;
preferably, said communicative connection between the medical device and the control device being disconnected, comprises: disconnecting, by the first control apparatus, the wireless connection between the first control apparatus and the control device; wherein the second control apparatus is configured to instruct the first control apparatus to disconnect the wireless connection, or the first control apparatus is configured to initiate to disconnect the wireless connection.

5. The control system according to claim 1, **characterized in that**, a communicative connection being established between the medical device and the control device, comprises: transmitting, by the first control apparatus, wireless connection information to the medical device, so as to enable the medical device to establish a wireless connection with the control device based on the wireless connection information; wherein the medical device is equipped with a wireless communication module, and the wireless connection information is transmitted from the second control apparatus to the first control apparatus, or the wireless connection information is pre-stored by the first control apparatus;
preferably, said communicative connection between the medical device and the control device being disconnected, comprises: instructing the medical device, by the first control apparatus, to disconnect the wireless connection with the control device.

6. The control system according to any one of claims 4-5, **characterized in that**, transmitting the wireless connection information to the first control apparatus, comprises: transmitting, by the second control apparatus, the wireless connection information to the first scanning module through the second scanning module.

7. The control system according to claim 1, **characterized in that**, in order for the position of the first control apparatus to change synchronously with the position of the medical device, the first control apparatus is integrated to the medical device; or
in order for the position of the first control apparatus to change synchronously with the position of the medical device, the position of the first control apparatus and the position of the medical device are associated, when the first control apparatus is an independent apparatus in the control system.

8. The control system according to claim 1, **characterized in that**, the second control apparatus is arranged on a ceiling of the operating room; or
the second control apparatus is an independent apparatus in the control system; or
the second control apparatus is integrated to the control device; or
the second control apparatus is integrated to a router of a wireless local area network where the control device is located, and the router is arranged inside the operating room;
the first scanning module and the second scanning module each comprises at least one of: a UWB module, a Wi-Fi module, an infrared module, and a Bluetooth module; or
the medical device comprises at least one of: a laparoscope, a pneumoperitoneum machine, a flushing suction pump, and an energy platform.

9. A control system for an operating room, **characterized in that**, comprising: a control device for the operating room, a first control apparatus, and a second control apparatus;
the first control apparatus is in communicative connection with a medical device, wherein the first control apparatus comprises a first scanning module, and a position of the first control apparatus changes synchronously with a position of the medical device;
the second control apparatus is arranged at a designated position of the operating room inside which room the control device is located, wherein the second control apparatus comprises a second scanning module; the first scanning module is configured to receive a scanning signal which is transmitted by the second scanning module and/or the second scanning module is configured to receive a scanning signal which is transmitted by the first scanning module, so as to obtain a scanning result;
wherein:
when the scanning result indicates that the first control apparatus is located inside a first area and no communicative connection has been established between the medical device and the control device, a communicative connection is established between the medical device and the control device; wherein the first area is an area, which is inside the operating room and capable of being scanned by the second control apparatus;
when the scanning result indicates that the first control apparatus is located outside the first area and no communicative connection has been established between the medical device and the control device, a communicative connection is not capable of being established between the medical device and the control device;
when a communicative connection has been established between the medical device and the control device, said communicative connection is maintained between the medical device and the control device until said communicative connection is disconnected, regardless of whether the scanning result indicates that the first control apparatus is located inside the first area or not.

10. A control method for a control device for an operating room, **characterized in that**, comprising:
obtaining position information of a medical device;
when the position information satisfies a first condition and no communicative connection has been established between the medical device and the control device, establishing a communicative connection between the medical device and the control device;
when the position information does not satisfy the first condition and no communicative connection has been established between the medical device and the control device, maintaining no communicative connection between the medical device and the control device;
when the position information satisfies the first condition or a second condition and a communicative connection has been established between the medical device and the control device, maintaining said communicative connection between the medical device and the control device;
when the position information does not satisfy the first condition and the second condition and a communicative connection has been established between the medical device and the control device, disconnecting said communicative connection between the medical device and the control device;
wherein an area which corresponds to a possible position of the medical device, which position satisfies the first condition, is less than an area which corresponds to a possible position of the medical device, which position satisfies the second condition.

11. The method according to claim 10, **characterized in that**, the position information satisfying the first condition indicates that the medical device is located inside a first area, which is inside the operating room;
the scanning result satisfying the second condition indicates that the medical device is located inside a second area, which covers the operating room entirely, or covers the first area and is greater than the first area.

12. A method for controlling connection of an operating room, which method is applicable to a control system for the operating room, **characterized in that**, the control system comprises a control device for the operating room, a first control apparatus, and a second control apparatus; wherein the first control apparatus is in communicative connection with a medical device, and a position of the first control apparatus changes synchronously with a position of the medical device; the second control apparatus is arranged at a designated position of the operating room inside which room the control device is located; wherein the method comprises:
obtaining a scanning result between the first control apparatus and the second control apparatus;
when the scanning result satisfies a first condition and no communicative connection has been established between the medical device and the control device, establishing a communicative connection between the medical device and the control device;
when the scanning result does not satisfy the first condition and no communicative connection has been established between the medical device and the control device, maintaining no communicative connection between the medical device and the control device;
when the scanning result satisfies the first condition or satisfies a second condition and a communicative connection has been established between the medical device and the control device, maintaining said communicative connection between the medical device and the control device;
when the scanning result does not satisfy the first condition and the second condition and a communicative connection has been established between the medical device and the control device, disconnecting said communicative connection between the medical device and the control device;
wherein, when the scanning result indicates a distance, a maximum distance which satisfies the first condition, is less than a maximum distance which satisfies the second condition; or
when the scanning result indicates a strength of a scanning signal, a minimum signal strength which satisfies the first condition is greater than a minimum signal strength which satisfies the second condition; or
when the scanning result indicates a position of the first control apparatus, an area which corresponds to a possible position of the first control apparatus, which position satisfies the first condition, is less than an area which corresponds to a possible position of the first control apparatus, which position satisfies the second condition.

13. The method according to claim 12, **characterized in that**, the scanning result satisfying the first condition indicates that a distance between the first control apparatus and the second control apparatus is less than or equal to a first distance; the scanning result satisfying the second condition indicates that the distance between the first control apparatus and the second control apparatus is less than or equal to a second distance; wherein the first distance is less than the second distance; or
the scanning result satisfying the first condition indicates that a distance between the first control apparatus and the second control apparatus is less than or equal to a first distance; the scanning result satisfying the second condition indicates that the distance between the first control apparatus and the second control apparatus is greater than a third distance but less than or equal to a second distance; wherein the first distance is less than the second distance, the third distance is not greater than the first distance; or
the scanning result satisfying the first condition indicates that a strength of the scanning signal is greater than or equal to a first signal strength; the scanning result satisfying the second condition indicates that the strength of the scanning signal is greater than or equal to a second signal strength; wherein the first signal strength is greater than the second signal strength; or
the scanning result satisfying the first condition indicates that a strength of the scanning signal is greater than or equal to a first signal strength; the scanning result satisfying the second condition indicates that the strength of the scanning signal is less than a third signal strength but greater than or equal to a second signal strength; wherein the third signal strength is not less than the first signal strength, the first signal strength is greater than the second signal strength.

14. The method according to claim 12, **characterized in that**, the scanning result satisfying the first condition indicates that the first control apparatus is located inside a first area which is inside the operating room and capable of being scanned by the second control apparatus;
the scanning result satisfying the second condition indicates that the first control apparatus is located inside a second area which is capable of being scanned by the second control apparatus; wherein the second area covers the operating room entirely, or the second area covers the first area and is greater than the first area.

15. The method according to claim 12, **characterized in that**,
establishing a communicative connection between the medical device and the control device, comprises: establishing, by the first control apparatus, a wireless connection between the control device and the first control apparatus, based on wireless connection information; and performing, by the first control apparatus, relay communication between the medical device and the control device; wherein the wireless connection information is transmitted from the second control apparatus to the first control apparatus, or is pre-stored by the first control apparatus; and disconnecting said communicative connection between the medical device and the control device, comprises: disconnecting, by the first control apparatus, the wireless connection between the first control apparatus and the control device; wherein the second control apparatus is configured to instruct the first control apparatus to disconnect the wireless connection, or the first control apparatus is configured to initiate to disconnect the wireless connection; or
establishing a communicative connection between the medical device and the control device, comprises: transmitting, by the first control apparatus, wireless connection information to the medical device, so as to enable the medical device to establish a wireless connection with the control device based on the wireless connection information; wherein the medical device is equipped with a wireless communication module, and the wireless connection information is transmitted from the second control apparatus to the first control apparatus, or the wireless connection information is pre-stored by the first control apparatus; and disconnecting said communicative connection between the medical device and the control device, comprises: instructing the medical device, by the first control apparatus, to disconnect the wireless connection with the control device.
